# EUROPEAN PATENT APPLICATION

(11) **EP 4 130 241 A1**
(43) Date of publication of application: **08.02.2023**
(21) Application number: 21779756.2
(22) Date of filing: 31.03.2021
(51) Int. Cl.: C12N 5/071, C12N 5/10

(54) **METHOD FOR SEPARATING PITUITARY HORMONE-PRODUCING CELLS AND PROGENITOR CELLS THEREOF**

(30) Priority: 31.03.2020 JP 2020065346
(71) Applicant: Fujita Academy, Toyoake-shi, Aichi 470-1192 (JP); National University Corporation Tokai National Higher Education and Research System, Nagoya-shi, Aichi 464-8601 (JP)
(72) Inventor: KODANI, Yu, Toyoake-shi, Aichi 470-1192 (JP); NAGASAKI, Hiroshi, Toyoake-shi, Aichi 470-1192 (JP); SUGA, Hidetaka, Nagoya-shi, Aichi 464-8601 (JP)
(74) Representative: J A Kemp LLP
(86) International application number: PCT/JP2021/014038
(87) International publication number: WO 2021/201175

(57) **Abstract**

The present invention provides a method for efficiently separating and purifying functional pituitary hormone-producing cells and/or progenitor cells thereof from differentiated tissues derived from pluripotent stem cells.

## Description

### [Technical Field]

The present invention relates to a method for separating and purifying, in vitro, pituitary hormone-producing cells induced to differentiate from pluripotent stem cells, and progenitor cells thereof.

### [Background Art]

The pituitary gland is a small endocrine organ that exists adjacent to the lower part of the diencephalon and plays a major role in the regulation of various hormones. For example, it produces various pituitary hormones including adrenocorticotropic hormone (ACTH), which promotes the production of adrenocortical hormones essential for life support, growth hormone, which promotes the growth of children, and the like. Therefore, pituitary dysfunction causes serious systemic diseases.

In recent years, a method for inducing differentiation of human ES/iPS cells into the adenohypophysis comprising functional pituitary hormone-producing cells has been developed, and its application to hypophyseal regenerative medicine is expected (Patent Literature 1, Non Patent Literatures 1 and 2). In these methods, a hypothalamic-pituitary composite tissue is formed in a cell aggregate by three-dimensional culture. In the composite tissue, the adenohypophysis and its precursor tissue are mainly located on the surface layer of the cell aggregate. By detaching and transplanting them to under the renal capsule of hypophysectomized mice, therapeutic effects such as improvement of activity, survival, body weight decrease and the like have been confirmed (Patent Literature 1, Non Patent Literature 1). However, a cell surface marker for isolating the adenohypophysis and precursor tissue thereof from the above-mentioned composite tissue is not known at all, and the discrimination of adenohypophysis and precursor tissue thereof to be detached relies on the subjective judgment made by experimenters based on morphology.

### [Citation List]

### [Patent Literature]

[PTL 1] WO 2016/013669

### [Non Patent Literature]

[NPL 1] Nature Communications, 7:10351, 2016
[NPL 2] Cell Reports, 30, 18-24, January 7, 2020

### [Summary of Invention]

### [Technical Problem]

The present inventors aim to provide an objective and reliable method for separating a cell of interest by utilizing a cell surface antigen for the purpose of separating pituitary hormone-producing cells and progenitor cells thereof.

### [Solution to Problem]

The present inventors first screened a panel of 332 human cell surface antigens in order to obtain surface antigens specific to the cell lineage of adenohypophysis. As a result, they identified surface antigens with high specificity for several types of pituitary hormone-producing cells and/or progenitor cells thereof, including epithelial cell adhesion molecule (hereinafter abbreviated as EpCAM). As a result of intensive studies, it was found that the identified surface antigen, EpCAM, can withstand treatments with enzymes or the like when dispersing cell aggregates of pluripotent stem cells, and that pituitary hormone-producing cells and progenitor cells thereof can be effectively separated and purified from the aggregates of pluripotent stem cells by sorting using an anti-EpCAM antibody. In addition, it was found that the separated and purified cells can be reaggregated and subjected to maintenance culture, that even after the maintenance culture, the cells exhibit superior pituitary hormone secretion ability by physiological stimulation of pituitary hormone secretion, and that the cells exhibit functions equivalent to those of the pituitary hormone-producing cells in the living body, which resulted in the completion of the present invention.

Accordingly, the present invention provides the following.
[1] A method for separating pituitary hormone-producing cells and/or progenitor cells thereof, comprising a step of separating cells that express EpCAM from a cell aggregate comprising adenohypophysis and/or a precursor tissue thereof.
[2] The method of [1], wherein the cell aggregate comprises a hypothalamic neuroepithelial tissue.
[3] The method of [1] or [2], comprising a step of dispersing the cell aggregate to obtain a population of single cells before the step of separating the cells that express EpCAM.
[4] The method of any one of [1] to [3], comprising a step of shredding the cell aggregate comprising adenohypophysis and/or a precursor tissue thereof, or physically incising the cell aggregate comprising adenohypophysis and/or a precursor tissue thereof before the step of separating the cells that express EpCAM.
[5] The method of any one of [1] to [4], wherein the cell aggregate comprising adenohypophysis and/or a precursor tissue thereof is obtained by inducing differentiation of pluripotent stem cells.
[6] The method of [5], wherein the aforementioned pluripotent stem cells are human induced pluripotent stem cells.
[7] The method of any one of [1] to [6], wherein the aforementioned precursor tissue is pituitary placode and/or Rathke's pouch.
[8] A method for producing pituitary hormone-producing cells and/or progenitor cells thereof, comprising a step of separating cells that express EpCAM from a cell aggregate comprising adenohypophysis and/or a precursor tissue thereof.
[9] The method of [8], comprising the following steps:
   (A) a step of dispersing a cell aggregate comprising adenohypophysis and/or a precursor tissue thereof to obtain a population of single cells,
   (B) a step of separating a population of cells that express EpCAM from the aforementioned population, and
   (C) a step of reaggregating the population obtained in the aforementioned (B).
[10] The method of [8] or [9], wherein the pituitary hormone-producing cells and/or progenitor cells thereof are of a cell aggregate or a cell sheet.
[11] The method of [9] or [10], comprising a step of shredding the cell aggregate comprising adenohypophysis and/or a precursor tissue thereof, or physically incising the cell aggregate comprising adenohypophysis and/or a precursor tissue thereof before the (B) step of separating the cells that express EpCAM.
[12] The method of any one of [8] to [11], wherein the aforementioned pituitary hormone-producing cell is at least one type selected from the group consisting of growth hormone (GH)-producing cells, prolactin (PRL)-producing cells, adrenocorticotropic hormone (ACTH)-producing cells, thyroid-stimulating hormone (TSH)-producing cells, follicle-stimulating hormone (FSH)-producing cells, and luteinizing hormone (LH)-producing cells.

### [Advantageous Effects of Invention]

According to the present invention, by utilizing EpCAM, functional pituitary hormone-producing cells and/or progenitor cells thereof can be efficiently separated and purified from differentiated tissues derived from pluripotent stem cells. The separated and purified pituitary hormone-producing cells exhibit superior pituitary hormone secreting ability by physiological stimulation of pituitary hormone secretion. Therefore, the diseases relating to pituitary gland can be treated using the cells.

### [Brief Description of Drawings]

[Fig. 1]

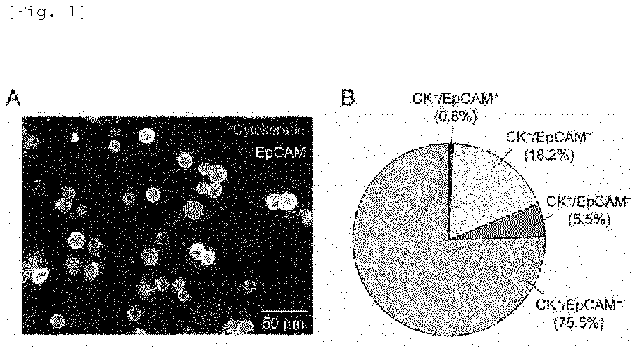

   Fig. 1 shows expression of EpCAM in pituitary progenitor cells derived from human iPS cells. The left figure shows an example in which human iPS cell aggregate on day 44 of differentiation induction were dispersed and subjected to fluorescent immunostaining with Cytokeratin (pituitary progenitor cell marker) and EpCAM (A). The right figure shows the quantification results of the expression rates of Cytokeratin (CK) and EpCAM in all cells on days 44 to 51 of differentiation induction (B). In the results, the numerical values show the mean of 4 experiments (B).
[Fig. 2]

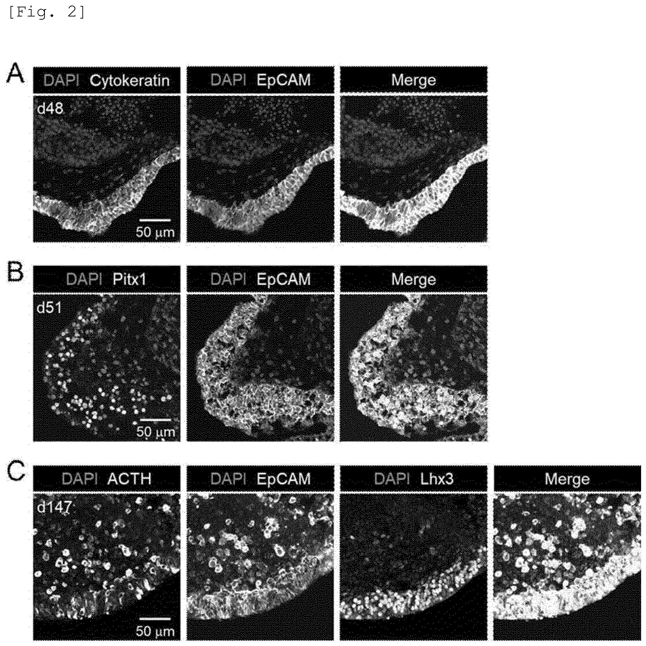

   Fig. 2 shows immunohistochemistry analysis of EpCAM in differentiated tissues derived from human iPS cells. The upper figure shows immunohistochemical staining of a human iPS cell aggregate on day 48 of differentiation induction (A). In the figure, EpCAM is expressed in Cytokeratin-positive pituitary progenitor cells (A). The central figure shows immunohistochemical staining of a human iPS cell aggregate on day 51 of differentiation induction (B). In the figure, EpCAM is expressed in Pitx1-positive pituitary progenitor cells (B). The lower figure shows immunohistochemical staining of a human iPS cell aggregate on day 147 of differentiation induction (C). In the figure, EpCAM is expressed in ACTH-positive ACTH-producing cells and Lhx3-positive pituitary progenitor cells (C) .
[Fig. 3]

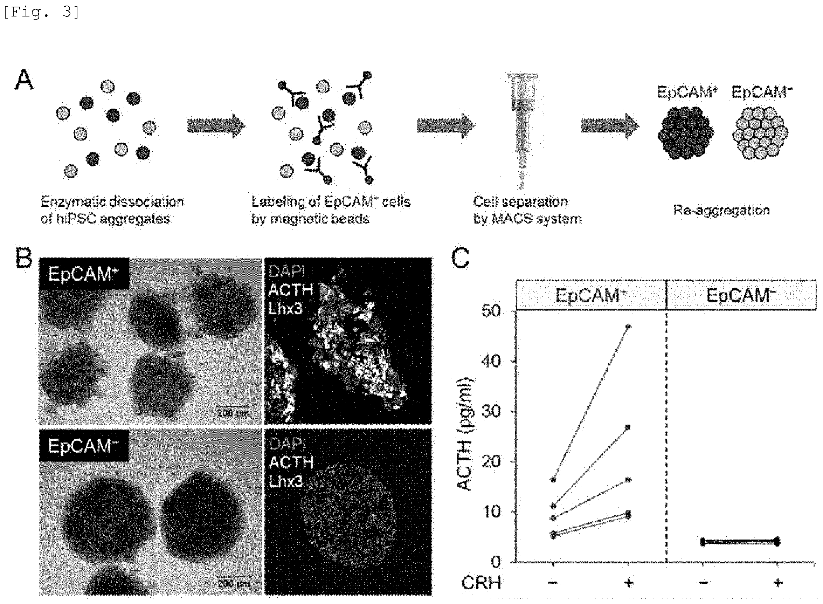

   Fig. 3 shows purification of ACTH-producing cells and pituitary progenitor cells by MACS using EpCAM as a marker. The upper figure shows the procedure of MACS (A). The lower left figure shows bright-field image (left) of reaggregated cell aggregates after MACS and an immunostaining image (right) of ACTH/Lhx3 of reaggregated cell aggregates after MACS (B). The lower right figure shows the amount of ACTH secreted by the reaggregated cell aggregates after MACS (C). In the experiment, the ACTH concentration in the culture supernatant was measured before and after the addition of CRH (C).

### [Description of Embodiments]

### (1) Pluripotent stem cell

The "pluripotent stem cell" refers to a cell that has both the ability to differentiate into all cells constituting the body (differentiation pluripotency) and the ability to produce daughter cells having the same differentiation potency as the self through cell division (self-renewal potential).

The differentiation pluripotency can be evaluated by transplanting the cells to be evaluated into nude mice and examining the presence or absence of teratoma formation containing cells of each of the three germ layers (ectoderm, mesoderm, endoderm).

Examples of the pluripotent stem cell include embryonic stem cells (ES cell), embryonic germ cells (EG cell), induced pluripotent stem cells (iPS cell), embryonal carcinoma cells (EC cell), and the like, and are not limited to these as long as the cell has both the differentiation pluripotency and the self-renewal potential. In the present invention, ES cell or iPS cell (more preferably human iPS cell) is preferably used. When the above-mentioned pluripotent stem cell is any cell derived from ES cell or human embryo, the cell may be a cell produced by destroying an embryo or without destroying an embryo, preferably a cell produced without destroying an embryo.

ES cell can be established by, for example, culturing early embryo before implantation, inner cell mass constituting the early embryo, single blastomere, and the like (Manipulating the Mouse Embryo A Laboratory Manual, Second Edition, Cold Spring Harbor Laboratory Press (1994), Thomson, J. A. et al., Science, 282, 1145-1147 (1998)). As the early embryo, an early embryo produced by nuclear transfer of the nucleus of a somatic cell may also be used (Wilmut et al., (Nature, 385, 810 (1997)), Cibelli et al. (Science, 280, 1256 (1998)), Akira Iritani et al. (protein nucleic acid enzyme, 44, 892 (1999)), Baguisi et al., (Nature Biotechnology, 17, 456 (1999)), Wakayama et al., (Nature, 394, 369 (1998); Nature Genetics, 22, 127 (1999); Proc. Natl. Acad. Sci. USA, 96, 14984 (1999)), Rideout III et al., (Nature Genetics, 24, 109 (2000)), Tachibana et al., (Human Embryonic Stem Cells Derived by Somatic Cell Nuclear Transfer, Cell (2013) in press)). As the early embryo, parthenogenetic embryo may also be used (Kim et al., (Science, 315, 482-486 (2007)), Nakajima et al., (Stem Cells, 25,983-985 (2007)), Kim et al., (Cell Stem Cell, 1, 346-352 (2007)), Revazova et al., (Cloning Stem Cells, 9, 432-44β (2007)), Revazova et al., (Cloning Stem Cells, 10, 11-24 (2008))).

Fused ES cells obtained by cell fusion of ES cells and somatic cells are also included in the ES cells to be used in the methods of the present invention.

ES cells can be obtained from designated institutions, and commercially available products can also be purchased. For example, human ES cells KhES-1, KhES-2, and ΣChES-3 are available from The Institute for Frontier Life and Medical Sciences, Kyoto University.

EG cells can be established by culturing primordial germ cells in the presence of LIF, bFGF, SCF, and the like (Matsui et al., Cell, 70, 841-847 (1992), Shamblott et al., Proc. Natl. Acad. Sci. USA, 95(23), 13726-13731 (1998), Turnpenny et al., Stem Cells, 21(5), 598-609, (2003)).

iPS cells refer to cells that have artificially acquired differentiation pluripotency and self-renewal potential by contacting somatic cells (e.g., fibroblast, skin cell, lymphocyte, etc.) with nuclear reprogramming factors. iPS cells were first discovered by a method of introducing nuclear reprogramming factors consisting of Oct3/4, Sox2, Klf4, and c-Myc into somatic cells (e.g., fibroblast, skin cell, etc.) (Cell, 126: p. 663-676, 2006). Since then, many researchers have made various improvements to combinations of reprogramming factors and methods of introducing factors, and various production methods of iPS cells have been reported.

The nuclear reprogramming factor may be configured with any substance, such as a proteinous factor or a nucleic acid that encodes the same (including a form integrated in a vector), or a low molecular compound, as long as it is a substance (substances) capable of inducing a cell having differentiation pluripotency and self-renewal potential from somatic cells such as fibroblast and the like. When the nuclear reprogramming factor is a proteinous factor or a nucleic acid that encodes the same, preferable nuclear reprogramming substance is exemplified by the following combinations (hereinafter, only the names for proteinous factors are shown).
(1) Oct3/4, Klf4, Sox2, c-Myc (here, Sox2 is replaceable with Soxl, Sox3, Sox15, Soxl7 or Sox18; Klf4 is replaceable with Klfl, Klf2 or KlfS; c-Myc is replaceable with T58A (active mutant), N-Myc or L-Myc)
(2) Oct3/4, Klf4, Sox2
(3) Oct3/4, Klf4, c-Myc
(4) Oct3/4, Sox2, Nanog, Lin28
(5) Oct3/4, Klf4, c-Myc, Sox2, Nanog, Lin28
(6) Oct3/4, Klf4, Sox2, bFGF
(7) Oct3/4, Klf4, Sox2, SCF
(8) Oct3/4, Klf4, c-Myc, Sox2, bFGF
(9) Oct3/4, Klf4, c-Myc, Sox2, SCF

Among these combinations, when the obtained iPS cell is to be used for therapeutic purposes, a combination of 3 factors of Oct3/4, Sox2 and Klf4 is preferred. On the other hand, when the iPS cell is not to be used for therapeutic purposes (e.g., used as an investigational tool for drug discovery screening and the like), 4 factors of Oct3/4, Klf4, Sox2, and c-Myc, or 5 factors of further including Lin28 or Nanog is preferred.

iPS cell is preferably used for autologous transplantation.

Pluripotent stem cells whose chromosomal genes have been altered using known genetic engineering techniques can also be used in the present invention. The pluripotent stem cell may be a cell that can be identified to have reached a corresponding differentiation stage, by using the expression of a marker gene (e.g., fluorescent protein such as GFP) as an index, which is achieved by knocking in the marker gene in-frame according to a known method to a gene encoding a differentiation marker.

As the pluripotent stem cells, pluripotent stem cells of, for example, warm-blooded animals, preferably mammals, can be used. Examples of the mammal include experimental animals such as rodents (e.g., mouse, rat, hamster, guinea pig, and the like), and rabbit and the like, domestic animals such as swine, bovine, goat, horse and sheep, pets such as dog and cat and the like, and the like, primates such as human, monkey, orangutan and chimpanzee, and the like, and the like. The pluripotent stem cell is preferably a pluripotent stem cell of rodents (mouse, rat, etc.) or primates (human, etc.), most preferably a human pluripotent stem cell.

Pluripotent stem cells can be maintenance cultured by a method known per se. For example, from the viewpoint of clinical application, pluripotent stem cells are preferably maintained by serum-free culture using a serum replacement such as Knockout^{™} Serum Replacement (KSR) and the like, or feeder-free cell culture.

Pluripotent stem cells used in the present invention are preferably isolated. The "isolation" means that an operation has been performed to remove factors other than the desired cells and components, and that the naturally occurring state has passed. The purity (percentage of human pluripotent stem cells to the total number of cells) of the "isolated human pluripotent stem cells" is generally 70% or more, preferably 80% or more, more preferably 90% or more, further preferably 99% or more, most preferably 100%.

### (2) Formation of pluripotent stem cell aggregate

The method for preparing aggregates of pluripotent stem cells is not particularly limited, and the dispersed pluripotent stem cells may be cultured under non-adhesive conditions (i.e., suspension culture) in a culture container, or may be cultured under adhesive conditions (i.e., adherent culture).

In one preferable embodiment, dispersed pluripotent stem cells are cultured in a culture vessel under non-adhesive conditions (i.e., suspension culture), and a plurality of pluripotent stem cells are assembled to form aggregates, whereby aggregates of pluripotent stem cells can be obtained.

A culture vessel used for the aggregate formation is not particularly limited, and, for example, flask, tissue culture flask, dish, petri dish, tissue culture dish, multidish, microplate, microwell plate, micropore, multiplate, multiwell plate, chamber slide, petri dish, tube, tray, culture bag, and roller bottle can be mentioned. To enable culture under non-adhesive conditions, the culture vessel is preferably non-adhesive to cells. As the cell non-adhesive culture vessel, a culture vessel whose surface has been artificially treated to be non-adhesive to cells, a culture vessel that has not been artificially treated (for example, coating treatment with extracellular matrix, etc.) for the purpose of improving adhesion to cells, or the like can be used.

The medium used for aggregate formation can be prepared using a medium used for culturing mammalian cells as a basal medium. Examples of the basal medium include BME medium, BGJb medium, CMRL 1066 medium, Glasgow MEM medium, Improved MEM Zinc Option medium, IMDM medium, medium 199 medium, Eagle MEM medium, αMEM medium, DMEM medium, Ham medium, Ham's F-12 medium, RPMI1640 medium, Fischer's medium, Neurobasal medium and a mixed medium of these (e.g., DMEM/F-12 medium (mixed medium of DMEM medium:Ham's F-12 medium=1:1) etc.) and the like, and the medium is not particularly limited as long as it can be used for culturing mammalian cells. In one embodiment, a mixed medium of IMDM medium and Ham's F-12 medium is used. The mixing ratio is, for example, IMDM:Ham's F-12=0.8 to 1.2:1.2 to 0.8 in volume ratio.

The medium used for culture may be a serum-containing medium or serum-free medium. The serum-free medium means a medium free of an unadjusted or unpurified serum, and a medium containing a purified blood-derived component or a purified animal tissue-derived component (e.g., growth factor) mixed therein is considered to fall under the serum-free medium. To avoid contamination with chemically undetermined components, a serum-free medium is preferably used in the present invention.

The medium used for aggregate formation may contain a serum replacement. The serum replacement may contain, for example, albumin, transferrin, fatty acid, collagen precursor, trace element, 2-mercaptoethanol or 3'thiolglycerol, or an equivalent of these and the like as appropriate. Such serum replacement can be prepared, for example, by the method described in WO 98/30679. To perform the method of the present invention more simply, a commercially available serum replacement can be utilized. Examples of such commercially available serum replacement include KSR (knockout serum replacement) (manufactured by Invitrogen), Chemically-defined Lipid concentrated (manufactured by Gibco), and Glutamax (manufactured by Gibco).

The medium used for aggregate formation may contain other additives as long as it does not adversely affect induction of differentiation of pluripotent stem cells into pituitary gland or a partial tissue thereof, or a precursor tissue thereof. Examples of the additive include, but are not limited to, insulin, iron source (e.g., transferrin, etc.), mineral (e.g., sodium selenite, etc.), saccharides (e.g., glucose, etc.), organic acid (e.g., pyruvic acid, lactic acid, etc.), serum protein (e.g., albumin, etc.), amino acid (e.g., L-glutamine, etc.), reducing agent (e.g., 2-mercaptoethanol, etc.), vitamins (e.g., ascorbic acid, d-biotin, etc.), antibiotic (e.g., streptomycin, penicillin, gentamicin, etc.), buffering agent (e.g., HEPES, etc.) and the like.

In addition, the medium used for aggregate formation may be the medium used in the first culture step, which is described later.

In the formation of pluripotent stem cell aggregates, pluripotent stem cells are first collected from the passage culture, and dispersed to a single cell state or a state close thereto. Dispersion of pluripotent stem cells is performed using an appropriate cell dissociation solution. As the cell dissociation solution, EDTA; trypsin, collagenase IV, protein degrading enzyme such as metalloprotease and the like, and the like can be used alone or in an appropriate combination. Among them, those with low cytotoxicity are preferred. As such cell dissociation solution, for example, commercially available products such as DISPASE (EIDIA Co., Ltd), TrypLE (Invitrogen), Accutase (MILLIPORE), and the like are available. The dispersed pluripotent stem cells are suspended in the above-mentioned medium.

In order to suppress cell death of pluripotent stem cells (particularly, human pluripotent stem cells) induced by dispersing, it is preferable to add an inhibitor of Rho-associated coiled-coil kinase (ROCK) from the start of culture (JP-A-2008-99662). A ROCK inhibitor is added, for example, within 15 days, preferably within 10 days, and more preferably within 6 days, from the start of culture. As the ROCK inhibitor, Y-27632((+)-(R)-trans-4-(1-aminoethyl)-N-(4-pyridyl)cyclohexanecarboxamide dihydrochloride) and the like can be mentioned. The concentration of the ROCK inhibitor used for suspension culture is a concentration that can suppress cell death of pluripotent stem cells induced by dispersing. For example, for Y-27632, such concentration is generally about 0.1 to 200 µM, preferably about 2 to 50 µM. The concentration of the ROCK inhibitor may be varied during the period of addition, and, for example, the concentration can be halved in the latter half of the period.

A suspension of the dispersed pluripotent stem cells is seeded in the above-mentioned culture vessel and the dispersed pluripotent stem cells are cultured in the culture vessel under non-adhesive conditions, whereby a plurality of pluripotent stem cells are assembled to form aggregates. At this time, by seeding the dispersed pluripotent stem cells in a relatively large culture vessel such as a 10 cm dish, multiple aggregates of pluripotent stem cells can be simultaneously formed in one culture compartment. In this case, however, the size of each aggregate and the number of pluripotent stem cells contained inside vary greatly. Due to the variation, the degree of differentiation from pluripotent stem cells into pituitary gland, a partial tissue thereof, or a precursor tissue thereof differs between aggregates, which in turn causes a decrease in the efficiency of differentiation induction. Therefore, it is preferable to rapidly coagulate dispersed pluripotent stem cells to form one aggregate in one culture compartment. As the methods for rapidly coagulating such dispersed pluripotent stem cells, for example, the following methods can be mentioned:
1) A method of confining dispersed pluripotent stem cells in a culture compartment of relatively small volume (e.g., 1 ml or less, 500 µl or less, 200 µl or less, 100 µl or less) and forming one aggregate in the compartment. Preferably, after confining the dispersed pluripotent stem cells, the culture compartment is left standing. Examples of the culture compartment include, but are not limited to, multiwell plates (384 wells, 192 wells, 96 wells, 48 wells, 24 wells, etc.), wells in micropore, chamber slide, and the like, tube, medium droplets in the hanging drop method, and the like. The dispersed pluripotent stem cells confined in the compartment are forced to deposit in one location by gravity, or adhere to each other to form one aggregate per one culture compartment. The shape of the bottom of multiwell plate, micropore, chamber slide, tube, and the like is preferably U-bottomed or V-bottomed so that the dispersed pluripotent stem cells can easily deposit in one place.
2) A method of placing the dispersed pluripotent stem cells in a centrifugation tube and centrifuging them to cause precipitation of the pluripotent stem cells in one location, thereby forming one aggregate in the tube.

The number of pluripotent stem cells to be seeded in one culture compartment is not particularly limited as long as one aggregate is formed per culture compartment, and the method of the present invention can induce the differentiation of pluripotent stem cells into pituitary gland or a partial tissue thereof, or a precursor tissue thereof in the aggregate. Generally about 1×10³ to about 5×10⁴, preferably about 1×10³ to about 2×10⁴, more preferably about 2×10³ to about 1.2×10⁴, pluripotent stem cells are seeded per culture compartment. By causing rapid coagulation of the pluripotent stem cells, one cell aggregate composed of generally about 1×10³ to about 5×10⁴, preferably about 1×10³ to about 2×10⁴, more preferably about 2×10³ to about 1.2×10⁴, pluripotent stem cells can be formed per culture compartment.

The time up to aggregate formation can be determined as appropriate within the range where one aggregate is formed per culture compartment, and the method of the present invention can induce the differentiation of pluripotent stem cells into pituitary gland or a partial tissue thereof, or a precursor tissue thereof in the aggregate. This time is preferably as short as possible because efficient induction of desired differentiation into pituitary gland or a partial tissue thereof, or a precursor tissue thereof can be expected by shortening this time. Preferably, aggregates of pluripotent stem cells are formed within 24 hours, more preferably within 12 hours, still more preferably within 6 hours, and most preferably within 2 to 3 hours. The time up to aggregate formation can be appropriately adjusted by those of ordinary skill in the art by adjusting tools for causing aggregation of cells, centrifugation conditions, and the like.

In addition, other culture conditions such as culture temperature, CO₂ concentration, and the like during aggregate formation can be appropriately set. The culture temperature is not particularly limited and is, for example, about 30 - 40°C, preferably about 37°C. The CO₂ concentration is, for example, about 1 to 10%, preferably about 5%.

Furthermore, by preparing a plurality of culture compartments under the same culture conditions and forming one aggregate of pluripotent stem cells in each culture compartment, a population of qualitatively uniform aggregates of pluripotent stem cells can be obtained. The qualitative uniformity of aggregates of pluripotent stem cells can be evaluated based on the size, the number of cells, macroscopic morphology, microscopic morphology and uniformity thereof by tissue staining analysis, expression of differentiation and undifferentiated markers and uniformity thereof, regulation of the expression of differentiation markers and synchronism thereof, of the aggregates, reproducibility of differentiation efficiency between aggregates, and the like. In one embodiment, the population of pluripotent stem cell aggregates used in the method of the present invention has a uniform number of pluripotent stem cells contained in the aggregates. Regarding a specific parameter, when a population of pluripotent stem cell aggregates is "uniform", it means that 90% or more of the aggregates in the entire aggregate population are within the range of mean±10%, preferably within the range of mean±5%, of the parameter in the aggregate population.

### (3) Induction of adenohypophysis and/or precursor tissue thereof

In the present invention, a cell aggregate containing the adenohypophysis and/or a precursor tissue thereof can be obtained by a method including suspension culturing the aggregates of pluripotent stem cells in a medium containing a bone morphogenic factor signal transduction pathway activating substance and a Sonic hedgehog (Shh) signal pathway agonist.

In the present invention, the adenohypophysis refers to a tissue containing at least one type of pituitary hormone-producing cell of the anterior or intermediate pituitary gland. Examples of the pituitary hormone-producing cell include cells constituting the anterior lobe such as growth hormone (GH)-producing cells, prolactin (PRL)-producing cells, adrenocorticotropic hormone (ACTH)-producing cells, thyroid-stimulating hormone (TSH)-producing cells, follicle-stimulating hormone (FSH)-producing cells, luteinizing hormone (LH)-producing cells, and the like; and cells constituting the intermediate lobe such as melanocyte stimulation hormone (MSH)-producing cells and the like. These pituitary hormone-producing cells can express EpCAM as a marker in addition to the expression of pituitary hormone specific to each cell type. In one embodiment, adenohypophysis contains at least one type, preferably two or more types (2, 3, 4, 5, or 6 types) of pituitary hormone-producing cells selected from the group consisting of GH-producing cells, PRL-producing cells, ACTH-producing cells, TSH-producing cells, FSH-producing cells, and LH-producing cells. In a further embodiment, adenohypophysis contains at least one type, preferably two types, more preferably 3 types, of pituitary hormone-producing cells selected from the group consisting of GH-producing cells, PRL-producing cells, and ACTH-producing cells.

In the present invention, the tissue refers to the structure of a cell population that has a structure in which multiple types of cells with different morphologies and properties are three-dimensionally configured in a certain pattern.

As the precursor tissue of adenohypophysis, pituitary placode, Rathke's pouch, and the like can be mentioned. The pituitary placode refers to a thickened structure that is formed in the region of the epidermal ectoderm (oral ectoderm) during embryogenesis and can express at least pituitary progenitor cell markers EpCAM, and Lhx3 or Pitxl, preferably all of EpCAM, Lhx3, and Pitxl. The Rathke's pouch refers to a sac-like structure formed by invagination of pituitary placode. The Rathke's pouch can also, like pituitary placode, express at least pituitary progenitor cell markers EpCAM, and Lhx3 or Pitxl, preferably all of EpCAM, Lhx3, and Pitxl. As pituitary progenitor cell markers other than the above, the expression of Isll/2, Cytokeratin, and the like may be further confirmed. In the present specification, a cell capable of expressing at least pituitary progenitor cell markers EpCAM, and Lhx3 or Pitxl is a pituitary progenitor cell, and this progenitor cell can preferably express all of EpCAM, Lhx3, and Pitxl. In the present specification, the pituitary progenitor cell is also referred to as a progenitor cell of pituitary hormone-producing cells.

As the "cell aggregate containing adenohypophysis and/or a precursor tissue thereof" in the present specification, for example, a cell aggregate positive for at least one marker selected from LHX3, NKX2.l, PITX1, and ACTH can be mentioned.

As the "cell aggregate containing adenohypophysis and/or a precursor tissue thereof", for example, a LHX3, NKX2.l, PITXl, and ACTH-positive cell aggregate can be mentioned.

The cell aggregate containing adenohypophysis and/or a precursor tissue thereof to be used in the separation method and the production method of the present invention can be produced by the following method. To be specific, the method includes suspension culturing pluripotent stem cell aggregates in a medium containing a bone morphogenic factor signal transduction pathway activation substance and an Shh signal pathway agonist to obtain cell aggregates containing hypothalamic neuroepithelial tissue and epidermal ectoderm (including oral ectoderm) (hereinafter sometimes to be simply abbreviated as epidermal ectoderm) (the first culture step), and further suspension culturing the obtained cell aggregates containing hypothalamic neuroepithelial tissue and epidermal ectoderm (including oral ectoderm) in a medium containing a bone morphogenic factor signal transduction pathway activation substance and an Shh signal pathway agonist to obtain cell aggregates containing 1) hypothalamic neuroepithelial tissue and 2) pituitary placode and/or Rathke's pouch (the second culture step). The first culture step induces differentiation of pluripotent stem cells into hypothalamic neuroepithelial tissue and epidermal ectoderm (including oral ectoderm), and further differentiation of epidermal ectoderm region (oral ectoderm) into pituitary placode and/or Rathke's pouch is induced by subjecting the obtained cell aggregates containing hypothalamic neuroepithelial tissue and epidermal ectoderm (including oral ectoderm) to the second culture step.

In the separation method and the production method of the present invention, the cell aggregate containing adenohypophysis and/or a precursor tissue thereof may be a cell aggregate obtained by the second culture step.

### (3.1) The first culture step

In the first culture step, pluripotent stem cell aggregates are cultured in suspension in a medium containing a bone morphogenic factor signal transduction pathway activation substance and a Shh signal pathway agonist.

That the pluripotent stem cell aggregates are "cultured in suspension" means that pluripotent stem cell aggregates are cultured in a medium under a condition of non-adhesive to the culture vessel. This enables efficient induction of adenohypophysis or a precursor tissue thereof, which has conventionally been difficult to achieve.

The medium used for suspension culture contains a bone morphogenic factor signal transduction pathway activation substance and a Shh signal pathway agonist. The differentiation of the pluripotent stem cells into hypothalamic neuroepithelial tissue and epidermal ectoderm is induced by the action of the bone morphogenic factor signal transduction pathway activation substance and the Shh signal pathway agonist.

In the present invention, the bone morphogenic factor signal transduction pathway activation substance means any substance that activates a pathway in which signals are transduced by the binding of a bone morphogenic factor and a receptor. Examples of the bone morphogenic factor signal transduction pathway activation substance include BMP2, BMP4, BMP7, GDF5, and the like. A preferred bone morphogenic factor signal transduction pathway activation substance is BMP4. While BMP4 is mainly described in the following, the bone morphogenic factor signal transduction pathway activation substance used in the present invention is not limited to BMP4. BMP4 is a known cytokine, and the amino acid sequence thereof is also known. The BMP4 used in the present invention is a mammalian BMP4. Examples of the mammal include rodents such as mouse, rat, hamster, guinea pig and the like, experimental animals such as rabbit and the like, domestic animals such as swine, bovine, goat, horse, sheep, and the like, pets such as dog, cat and the like, primates such as human, monkey, orangutan, chimpanzee, and the like, and the like. BMP4 is preferably a BMP4 of rodents (mouse, rat etc.) or primates (human etc.), most preferably human BMP4. Human BMP4 means that BMP4 has the amino acid sequence of BMP4 that humans naturally express in vivo. Representative amino acid sequences of human BMP4 are, for example, NCBI accession numbers NP_001193.2 (updated June 15, 2013), NP_570911.2 (updated June 15, 2013), and NP_570912.2 (updated June 15, 2013), amino acid sequences (mature human BMP4 amino acid sequences) obtained by removing N terminal signal sequence (1-24) from each of these amino acid sequences, and the like.

In the present invention, the Shh signal pathway agonist is not particularly limited as long as it can enhance signal transduction mediated by Shh. Examples of the Shh signal pathway agonist include, but are not limited to, proteins belonging to the Hedgehog family and fragments thereof (e.g., Shh, Ihh, Shh(C24II) N-Terminal, Shh(C25II) N-Terminal), Shh receptor, Shh receptor agonist, Purmorphamine, Smoothened - Agonist (SAG) (3-Chloro-N-[trans-4-(methylamino)cyclohexyl]-N-[[3-(4-pyridinyl)phenyl]methyl]-benzo[b]thiophene-2-carboxamide), and the like. Among these, SAG is preferred.

A preferred combination of the bone morphogenic factor signal transduction pathway activation substance and the Shh signal pathway agonist is BMP4 and SAG.

The concentration of the bone morphogenic factor signal transduction pathway activation substance in the medium can be appropriately determined within the range where differentiation from pluripotent stem cells to hypothalamic neuroepithelial tissue and epidermal ectoderm can be induced. When BMP4 is used as the bone morphogenic factor signal transduction pathway activation substance, its concentration is generally 0.01 nM or more, preferably 0.1 nM or more, more preferably 1 nM or more. The upper limit is not particularly set as long as differentiation into hypothalamic neuroepithelial tissue and epidermal ectoderm is not adversely affected. From the aspect of culture cost, it is generally 1000 nM or less, preferably 100 nM or less, more preferably 10 nM or less. In one embodiment, the concentration of BMP4 in the medium is generally 0.01 to 1000 nM, preferably 0.1 to 100 nM, more preferably 1 to 10 nM (e.g., 5 nM). Exogenous bone morphogenic factor signal transduction pathway activation substance particularly contributes to 1) active formation of epidermal ectoderm and 2) induction of differentiation into neuroepithelial tissue of the hypothalamus, not of the cerebrum, in cell aggregates. Thus, it is contained in the medium at concentrations capable of achieving these effects.

The bone morphogenic factor signal transduction pathway activation substance may not be contained in the medium for the entire period of the first culture step. For example, the bone morphogenic factor signal transduction pathway activation substance may not be added to the medium 2 to 4 days (e.g., 3 days) from the start of the suspension culture of pluripotent stem cell aggregates, and thereafter the bone morphogenic factor signal transduction pathway activation substance may be added to the medium.

The concentration of the Shh signal pathway agonist in the medium can be appropriately determined within the range where differentiation from pluripotent stem cells to hypothalamic neuroepithelial tissue and epidermal ectoderm can be induced. When SAG is used as the Shh signal pathway agonist, its concentration is generally 1 nM or more, preferably 10 nM or more, more preferably 100 nM or more. The upper limit is not particularly set as long as differentiation into hypothalamic neuroepithelial tissue and epidermal ectoderm is not adversely affected. From the aspect of culture cost, it is generally 1000 µM or less, preferably 100 µM or less, more preferably 10 µM or less. In one embodiment, the concentration of SAG in the medium is generally 1 nM to 1000 µM, preferably 10 nM to 100 µM, more preferably 100 nM to 10 µM (e.g., 2 µM). Exogenous Shh signal pathway agonist particularly contributes to the induction of differentiation into neuroepithelial tissue of the hypothalamus (preferably ventral hypothalamus), not of the neural retina, in cell aggregates. Thus, it is contained in the medium at a concentration capable of achieving this effect.

The Shh signal pathway agonist may not be contained in the medium for the entire period of the first culture step. For example, the Shh signal pathway agonist may not be added to the medium 5 to 7 days (e.g., 6 days) from the start of the suspension culture of pluripotent stem cell aggregates, and thereafter the Shh signal pathway agonist may be added to the medium.

In one embodiment, pluripotent stem cell aggregates are cultured in suspension for 2 to 4 days in a medium not containing a bone morphogenic factor signal transduction pathway activation substance or an Shh signal pathway agonist, then the obtained aggregates are cultured in suspension for 2 to 4 days in a medium containing a bone morphogenic factor signal transduction pathway activation substance but not containing an Shh signal pathway agonist, and further the obtained aggregates are cultured in suspension in a medium containing a bone morphogenic factor signal transduction pathway activation substance and an Shh signal pathway agonist until hypothalamic neuroepithelial tissue and epidermal ectoderm are induced.

Bone morphogenic factor signal transduction pathway activation substances such as BMP4 and the like used in the present invention are preferably isolated. The "isolation" means that an operation has been performed to remove factors other than the desired components and cells, and that the naturally occurring state has passed. Therefore, the "isolated protein X" does not include endogenous protein X produced from a cell or tissue to be cultured. The purity (percentage of weight of protein X to the total protein weight) of the "isolated protein X" is generally 70% or more, preferably 80% or more, more preferably 90% or more, further preferably 99% or more, most preferably 100%. The isolated bone morphogenic factor signal transduction pathway activation substance contained in the medium used for suspension culture is exogenously added to the medium. Therefore, in one embodiment, the present invention includes a step of exogenously adding an isolated bone morphogenetic factor signal transduction pathway activation substance to the medium used in the first culture step.

In order to suppress cell death of pluripotent stem cells (particularly, human pluripotent stem cells) induced by dispersing, it is preferable to add an inhibitor of Rho-associated coiled-coil kinase (ROCK) to the medium to be used in the first culture step from the start of culture (JP-A-2008-99662). A ROCK inhibitor is added, for example, within 15 days, preferably within 10 days, and more preferably within 6 days, from the start of culture. As the ROCK inhibitor, Y-27632((+)-(R)-trans-4-(1-aminoethyl)-N-(4-pyridyl)cyclohexanecarboxamide dihydrochloride) and the like can be mentioned. The concentration of the ROCK inhibitor used for suspension culture is a concentration that can suppress cell death of pluripotent stem cells induced by dispersing. For example, for Y-27632, such concentration is generally about 0.1 to 200 µM, preferably about 2 to 50 µM. The concentration of the ROCK inhibitor may be varied during the period of addition, and, for example, the concentration can be halved in the latter half of the period.

The medium used for suspension culture of cell aggregates can be prepared using a medium used for culturing mammalian cells as a basal medium. Examples of the basal medium include BME medium, BGJb medium, CMRL 1066 medium, Glasgow MEM medium, Improved MEM Zinc Option medium, IMDM medium, medium 199 medium, Eagle MEM medium, αMEM medium, DMEM medium, Ham medium, Ham's F-12 medium, RPMI1640 medium, Fischer's medium, Neurobasal medium and a mixed medium of these (e.g., DMEM/F-12 medium (mixed medium of DMEM medium:Ham's F-12 medium-1:1) etc.) and the like, and the medium is not particularly limited as long as it can be used for culturing mammalian cells. In one embodiment, a mixed medium of IMDM medium and Ham's F-12 medium is used. The mixing ratio is, for example, IMDM:Ham's F-12=0.8 to 1.2:1.2 to 0.8 in volume ratio.

The medium used for culture may be a serum-containing medium or serum-free medium. To avoid contamination with chemically undetermined components, the medium to be used for the suspension culture of cell aggregates is preferably a serum-free medium.

The medium used for suspension culture of cell aggregates may contain a serum replacement. The serum replacement may contain, for example, albumin, transferrin, fatty acid, collagen precursor, trace element, 2-mercaptoethanol or 3'thiolglycerol, or an equivalent of these and the like as appropriate. Such serum replacement can be prepared, for example, by the method described in WO 98/30679. To perform the method of the present invention more simply, a commercially available serum replacement can be utilized. Examples of such commercially available serum replacement include KSR (knockout serum replacement) (manufactured by Invitrogen), Chemically-defined Lipid concentrated (manufactured by Gibco), and Glutamax (manufactured by Gibco).

The medium used for suspension culture of cell aggregates may contain other additives as long as it does not adversely affect induction of differentiation of pluripotent stem cells into hypothalamic neuroepithelial tissue and epidermal ectoderm. Examples of the additive include, but are not limited to, insulin, iron source (e.g., transferrin, etc.), mineral (e.g., sodium selenite, etc.), saccharides (e.g., glucose, etc.), organic acid (e.g., pyruvic acid, lactic acid, etc.), serum protein (e.g., albumin, etc.), amino acid (e.g., L-glutamine, etc.), reducing agent (e.g., 2-mercaptoethanol, etc.), vitamins (e.g., ascorbic acid, d-biotin, etc.), antibiotic (e.g., streptomycin, penicillin, gentamicin, etc.), buffering agent (e.g., HEPES, etc.) and the like.

In one embodiment, from the viewpoint of not adversely affecting the induction of differentiation into hypothalamic neuroepithelial tissue and epidermal ectoderm, the medium used for suspension culture of cell aggregates is a chemically defined medium that does not contain growth factors other than those particularly described in the present specification to be contained in the medium (growth-factor-free Chemically Defined medium; gfCDM) and is supplemented with a serum replacement (KSR etc.). The "growth factor" here includes Fgf; BMP; pattern formation factors such as Wnt, Nodal, Notch, Shh, and the like; and insulin and Lipid-rich albumin. As the chemically defined medium not containing a growth factor, for example, gfCDM disclosed in Wataya et al, Proc Natl Acad Sci USA, 105(33): 11796-11801, 2008 can be mentioned.

Other culture conditions such as culture temperature, CO₂ concentration, O₂ concentration, and the like in suspension culture of cell aggregates can be appropriately set. The culture temperature is, for example, about 30 - 40°C, preferably about 37°C. The CO₂ concentration is, for example, about 1 to 10%, preferably about 5%. The O₂ concentration is, for example, about 20%.

In a preferred embodiment, a population of qualitatively uniform aggregates of pluripotent stem cells is cultured in suspension in a medium containing a bone morphogenic factor signal transduction pathway activation substance and an Shh signal pathway agonist. Using a population of qualitatively uniform aggregates of pluripotent stem cells, the difference in the degree of differentiation into adenohypophysis or a precursor tissue thereof between aggregates can be minimized and the efficiency of the desired differentiation induction can be improved. The suspension culture of a population of qualitatively uniform aggregates of pluripotent stem cells includes the following embodiments.
1) A plurality of culture compartments are prepared and a population of qualitatively uniform aggregates of pluripotent stem cells is seeded such that one pluripotent stem cell aggregate is contained in one culture compartment (e.g., one pluripotent stem cell aggregate is placed in each well of 96 well plate). Then, in each culture compartment, one pluripotent stem cell aggregate is cultured in suspension in a medium containing a bone morphogenic factor signal transduction pathway activation substance and a Shh signal pathway agonist.
2) Populations of qualitatively uniform aggregates of pluripotent stem cells are seeded in one culture compartment such that a plurality of pluripotent stem cell aggregates are contained in one culture compartment (e.g., plural pluripotent stem cell aggregates are placed in a 10 cm dish). Then, in the culture compartment, a plurality of pluripotent stem cell aggregates are cultured in suspension in a medium containing a bone morphogenic factor signal transduction pathway activation substance and an Shh signal pathway agonist.

Throughout the method of the present invention, any embodiment of the above-mentioned 1) and 2) may be adopted, and the embodiment may be changed during the culture. <From embodiment 1) to embodiment 2), or from embodiment 2) to embodiment 1)>. In one embodiment, the first culture step employs the embodiment 1), and the second culture step employs the embodiment 2).

The first culture step is conducted for a period of time sufficient to induce differentiation of pluripotent stem cells into hypothalamic neuroepithelial tissue and epidermal ectoderm. The differentiation of pluripotent stem cells into hypothalamic neuroepithelial tissue and epidermal ectoderm can be detected by, for example, RT-PCR and immunohistochemistry using marker-specific antibodies in hypothalamic neuroepithelial tissue and epidermal ectoderm. For example, the step is conducted until 10% or more, preferably 30% or more, more preferably 50% or more, of the cell aggregates in the culture contain hypothalamic neuroepithelial tissue and epidermal ectoderm. The culture period may vary according to the animal species of the pluripotent stem cells, and the kind of the bone morphogenic factor signal transduction pathway activation substance and the Shh signal pathway agonist and cannot be specified. When a human pluripotent stem cell is used, the first culture step is, for example, generally 15 to 20 days (e.g., 18 days).

By performing the first culture step, cell aggregates containing hypothalamic neuroepithelial tissue and epidermal ectoderm (including oral ectoderm) can be obtained.

The hypothalamic neuroepithelial tissue refers to a neuroepithelial tissue that expresses a hypothalamic marker, can differentiate into a cell constituting hypothalamus (e.g., neuron, glial cell, and the like), and contains a hypothalamic progenitor cell having self-renewal potential, neurons and glial cells that have lost self-renewal potential due to differentiation, and the like. The ratio of hypothalamic progenitor cells and differentiated cells thereof in the hypothalamic neuroepithelial tissue varies depending on the degree of differentiation. It has been confirmed that many of the hypothalamic progenitor cells differentiate into hypothalamic neurons in cell aggregates obtained by culturing pluripotent stem cells for a long term until they differentiate into adenohypophysis (Cell Reports, 30, 18-24, January 7, 2020). The hypothalamus includes ventral hypothalamus and dorsal hypothalamus. As the hypothalamic marker, NKx2.1 (ventral hypothalamic marker), Pax6 (dorsal hypothalamic marker), and the like can be mentioned. In one embodiment, the ventral hypothalamic neuroepithelial tissue is an Rx-positive, Chx10-negative, Pax6-negative, and Nkx2.1-positive neuroepithelial tissue. In one embodiment, the dorsal hypothalamic neuroepithelial tissue is an Rx-positive, Chx10-negative, Nkx2.1-negative, and Pax6-positive neuroepithelial tissue. The hypothalamic neuroepithelial tissue contained in the cell aggregate obtained in the first culture step is preferably a ventral hypothalamic neuroepithelial tissue. EpCAM is not expressed in any of the above-mentioned hypothalamic neuroepithelial tissues, which means that EpCAM is not expressed in all cells that constitute the hypothalamic neuroepithelial tissue (not only hypothalamic progenitor cells, but also differentiated neurons and glial cells that have lost self-renewal potential).

The epidermal ectoderm is an ectodermal cell layer formed on the surface layer of the embryo during embryogenesis. As an epidermal ectoderm marker, pan-cytokeratin can be mentioned. The epidermal ectoderm can be generally differentiated into anterior pituitary gland, skin, oral epithelium, tooth enamel, dermal gland, and the like. In one embodiment, the epidermal ectoderm is an E-cadherin-positive and pan-cytokeratin-positive cell layer.

Preferably, in the cell aggregates obtained in the first culture step, the hypothalamic neuroepithelial tissue occupies the interior of the cell aggregates, and a single layer of epidermal ectodermal cells constitutes the surface of the cell aggregates. The epidermal ectoderm may contain a thickened epidermal placode in a part thereof.

### (3.2) the second culture step

In the second culture step, the cell aggregates obtained in the first culture step and containing hypothalamic neuroepithelial tissue and epidermal ectoderm (including oral ectoderm) are further cultured in suspension in a medium containing a bone morphogenic factor signal transduction pathway activation substance and a Shh signal pathway agonist to give cell aggregates containing 1) hypothalamic neuroepithelial tissue, and 2) pituitary placode and/or Rathke's pouch. By the action of the bone morphogenic factor signal transduction pathway activation substance and the Shh signal pathway agonist, the differentiation of epidermal ectoderm into pituitary placode and/or Rathke's pouch is induced.

The definitions of the bone morphogenic factor signal transduction pathway activation substance and the Shh signal pathway agonist are as described in the first culture step.

Preferably, the bone morphogenic factor signal transduction pathway activation substance to be used in the second culture step is the same as that in the first culture step and is BMP4. Preferably, the Shh signal pathway agonist to be used in the second culture step is the same as that in the first culture step and is SAG.

A preferred combination of the bone morphogenic factor signal transduction pathway activation substance and the Shh signal pathway agonist is BMP4 and SAG.

The concentration of the bone morphogenic factor signal transduction pathway activation substance in the medium can be appropriately determined within the range where differentiation from epidermal ectoderm to pituitary placode and/or Rathke's pouch can be induced. When BMP4 is used as the bone morphogenic factor signal transduction pathway activation substance, its concentration is generally 0.01 nM or more, preferably 0.1 nM or more, more preferably 1 nM or more. The upper limit is not particularly set as long as differentiation of epidermal ectoderm into pituitary placode and/or Rathke's pouch is not adversely affected. From the aspect of culture cost, it is generally 1000 nM or less, preferably 100 nM or less, more preferably 10 nM or less. In one embodiment, the concentration of BMP4 in the medium is generally 0.01 to 1000 nM, preferably 0.1 to 100 nM, more preferably 1 to 10 nM (e.g., 5 nM). The concentration of the bone morphogenic factor signal transduction pathway activation substance may be varied during the period of addition. For example, the aforementioned concentration may be used at the start of the second culture step, and the concentration may be reduced stepwise to half every 2 to 4 days.

The concentration of the Shh signal pathway agonist in the medium can be appropriately determined within the range where differentiation from epidermal ectoderm into pituitary placode and/or Rathke's pouch can be induced. When SAG is used as the Shh signal pathway agonist, its concentration is generally 1 nM or more, preferably 10 nM or more, more preferably 100 nM or more. The upper limit is not particularly set as long as differentiation into pituitary placode and/or Rathke's pouch is not adversely affected. From the aspect of culture cost, it is generally 1000 µM or less, preferably 100 µM or less, more preferably 10 µM or less. In one embodiment, the concentration of SAG in the medium is generally 1 nM to 1000 µM, preferably 10 nM to 100 µM, more preferably 100 nM to 10 µM (e.g., 2 µM).

In a preferred embodiment, the medium used in the second culture step contains FGF2. FGF2 promotes differentiation of epidermal ectoderm into pituitary placode.

FGF2 is a known cytokine also called a basic fibroblast growth factor (bFGF), and the amino acid sequence thereof is also known. The FGF2 used in the present invention is generally a mammalian FGF2. As the mammal, those mentioned above can be recited. Since FGF2 has cross-reactivity among many mammalian species, any mammalian FGF2 may be used as long as the purpose of the present invention can be achieved. Preferably, mammalian FGF2 of the same species as the cells to be cultured is used. For example, FGF2 of rodents (mouse, rat, etc.) or primates (human, etc.) is used. As used herein, mouse FGF2 means that FGF2 has the amino acid sequence of FGF2 that mice naturally express in vivo. Other protein and the like in the present specification are also interpreted similarly. Representative amino acid sequences of mouse FGF2 are, for example, NCBI accession number NP_032032.1 (updated February 18, 2014), an amino acid sequence (mature mouse FGF2 amino acid sequences) obtained by removing N terminal signal sequence (1-9) from this amino acid sequence, and the like. Representative amino acid sequences of human FGF2 are, for example, NCBI accession number NP_001997.5 (updated February 18, 2014) and the like.

The concentration of FGF2 in the medium is not particularly limited as long as it can promote differentiation of epidermal ectoderm into pituitary placode. It is generally 1 ng/ml or more, preferably 10 ng/ml or more. The upper limit of the FGF2 concentration is not particularly set as long as differentiation into pituitary placode and/or Rathke's pouch is not adversely affected. From the aspect of culture cost, it is generally 1000 ng/ml or less, preferably 500 ng/ml or less. In one embodiment, the concentration of FGF2 in the medium is generally 1 to 1000 ng/ml, preferably 10 to 100 ng/ml.

Bone morphogenic factor signal transduction pathway activation substance such as BMP4 and the like and FGF2 used in the present invention are preferably isolated. The isolated bone morphogenic factor signal transduction pathway activation substance and isolated FGF2 contained in the medium used in the second culture step are exogenously added to the medium. Therefore, in one embodiment, the present invention includes a step of exogenously adding an isolated bone morphogenetic factor signal transduction pathway activation substance (and optionally isolated FGF2) to the medium used in the second culture step.

The medium used in the second culture step can be prepared using a medium used for culturing mammalian cells as a basal medium, similar to the medium used in the first culture step. Examples of the basal medium include BME medium, BGJb medium, CMRL 1066 medium, Glasgow MEM medium, Improved MEM Zinc Option medium, IMDM medium, medium 199 medium, Eagle MEM medium, αMEM medium, DMEM medium, ham medium, Ham's F-12 medium, RPMI1640 medium, Fischer's medium, Neurobasal medium and a mixed medium of these (e.g., DMEM/F-12 medium (mixed medium of DMEM medium:Ham's F-12 medium=1:1) etc.) and the like, and the medium is not particularly limited as long as it can be used for culturing mammalian cells. In one embodiment, a mixed medium of IMDM medium and Ham's F-12 medium is used. The mixing ratio is, for example, IMDM:Ham's F-12=0.8 to 1.2:1.2 to 0.8 in volume ratio.

The medium used for culture may be a serum-containing medium or serum-free medium. To avoid contamination with chemically undetermined components, the medium to be used for the suspension culture of cell aggregates is preferably a serum-free medium.

The medium used for suspension culture of cell aggregates may contain a serum replacement. The serum replacement may contain, for example, albumin, transferrin, fatty acid, collagen precursor, trace element, 2-mercaptoethanol or 3'thiolglycerol, or an equivalent of these and the like as appropriate. Such serum replacement can be prepared, for example, by the method described in WO 98/30679. To perform the method of the present invention more simply, a commercially available serum replacement can be utilized. Examples of such commercially available serum replacement include KSR (knockout serum replacement) (manufactured by Invitrogen), Chemically-defined Lipid concentrated (manufactured by Gibco), and Glutamax (manufactured by Gibco).

The medium used for suspension culture of cell aggregates may contain other additives as long as it does not adversely affect induction of differentiation of epidermal ectoderm into pituitary placode and/or Rathke's pouch. Examples of the additive include, but are not limited to, insulin, iron source (e.g., transferrin, etc.), mineral (e.g., sodium selenite, etc.), saccharides (e.g., glucose, etc.), organic acid (e.g., pyruvic acid, lactic acid, etc.), serum protein (e.g., albumin, etc.), amino acid (e.g., L-glutamine, etc.), reducing agent (e.g., 2-mercaptoethanol, etc.), vitamins (e.g., ascorbic acid, d-biotin, etc.), antibiotic (e.g., streptomycin, penicillin, gentamicin, etc.), buffering agent (e.g., HEPES, etc.) and the like.

In one embodiment, from the viewpoint of not adversely affecting the induction of differentiation into pituitary placode and/or Rathke's pouch, the medium used for suspension culture of cell aggregates is a chemically defined medium that does not contain growth factors other than those particularly described in the present specification to be contained in the medium (growth-factor-free Chemically Defined medium; gfCDM) and is supplemented with a serum replacement (KSR etc.). The "growth factor" here includes Fgf; BMP; pattern formation factors such as Wnt, Nodal, Notch, Shh, and the like; and insulin and Lipid-rich albumin. As the chemically defined medium not containing a growth factor, for example, gfCDM disclosed in Wataya et al, Proc Natl Acad Sci USA, 105(33): 11796-11801, 2008 can be mentioned.

The suspension culture in the second culture step is preferably performed under high oxygen partial pressure conditions. Further suspension culture of cell aggregates containing hypothalamic neuroepithelial tissue and epidermal ectoderm under high oxygen partial pressure conditions allows oxygen to reach the inside of the cell aggregates and achieves maintenance culture of the cell aggregates for a long period of time, which in turn enables efficient induction of differentiation into pituitary placode and/or Rathke's pouch.

The high oxygen partial pressure conditions mean oxygen partial pressure conditions above the oxygen partial pressure in air (20%). The oxygen partial pressure in the second culture step is, for example, 30 to 60%, preferably 35 to 60%, more preferably 38 to 60%.

Other culture conditions such as culture temperature, CO₂ concentration and the like in the second culture step can be appropriately set. The culture temperature is, for example, about 30 to 40°C, preferably about 37°C. The CO₂ concentration is, for example, about 1 to 10%, preferably about 5%.

The second culture step is conducted for a period of time sufficient to induce differentiation of epidermal ectoderm into pituitary placode and/or Rathke's pouch. By performing the second culture step, pituitary placode can be formed in the epidermal ectoderm (more specifically, in the oral ectoderm). In addition, a part or all of the pituitary placode invaginates toward the inside of the cell aggregate (i.e., adjacent hypothalamic neuroepithelium) to optionally form Rathke's pouch. Differentiation of epidermal ectoderm into pituitary placode and/or Rathke's pouch essentially requires interaction between epidermal ectoderm and hypothalamic neuroepithelial tissue (preferably, ventral hypothalamic neuroepithelial tissue). In the present invention, hypothalamic neuroepithelial tissue and epidermal ectoderm are simultaneously formed in the cell aggregates in the first culture step, hypothalamic neuroepithelial tissue occupies the inside of the cell aggregates in a preferred embodiment, and the cells of the epidermal ectoderm in a single layer constitute the surface of the cell aggregates. As a result, a good interaction between the adjacent epidermal ectoderm and hypothalamic neuroepithelial tissue becomes possible in the cell aggregates, and the process of self organization of pituitary gland during embryonic development, such as formation of pituitary placode in epidermal ectoderm, invagination of pituitary placode, formation of Rathke's pouch, and the like can be reproduced in vitro. Differentiation into pituitary placode and/or Rathke's pouch can be confirmed, for example, by detecting the formation of a pituitary progenitor cell marker-positive placode or pouch-like structure, by immunohistochemistry using specific antibodies against pituitary progenitor cell markers (e.g. EpCAM, Pitx1, Lhx3, etc.). For example, the second culture step is conducted until 10% or more, preferably 30% or more, more preferably 50% or more, of the cell aggregates in the culture contain pituitary placode and/or Rathke's pouch. The culture period may vary according to the animal species of the pluripotent stem cells, and the kind of the bone morphogenic factor signal transduction pathway activation substance and the Shh signal pathway agonist and cannot be specified. When a human pluripotent stem cell is used, the period of the second culture step is, for example, generally not less than 6 days, for example, 6 to 12 days.

By performing the second culture step, cell aggregates containing 1) hypothalamic neuroepithelial tissue, and 2) pituitary placode and/or Rathke's pouch can be obtained. In the separation method and production method of the present invention, the cell aggregate containing adenohypophysis and/or a precursor tissue thereof may be a cell aggregate obtained in the second culture step.

### (3.3) The third culture step

Further suspension culture of the cell aggregates containing 1)hypothalamic neuroepithelial tissue, and 2)pituitary placode and/or Rathke's pouch, obtained in the second culture step, in a medium containing an Shh signal pathway agonist affords cell aggregates containing adenohypophysis (third culture step). By the third culture step, differentiation of pituitary placode and/or Rathke's pouch into pituitary hormone-producing cells is induced, pituitary hormone-producing cells are produced in the pituitary placode and/or Rathke's pouch, and adenohypophysis can be formed.

In the separation method and the production method of the present invention, the cell aggregate containing adenohypophysis and/or a precursor tissue thereof may be a cell aggregate obtained by the third culture step.

The definitions of the Shh signal pathway agonist are as described in the first culture step.

Preferably, the Shh signal pathway agonist to be used in the third culture step is the same as that in the first and the second culture steps and is SAG.

The concentration of the Shh signal pathway agonist in the medium can be appropriately determined within the range where differentiation from pituitary placode and/or Rathke's pouch into pituitary hormone-producing cells can be induced. When SAG is used as the Shh signal pathway agonist, its concentration is generally 1 nM or more, preferably 10 nM or more, more preferably 100 nM or more. The upper limit is not particularly set as long as differentiation into pituitary hormone-producing cells is not adversely affected. From the aspect of culture cost, it is generally 1000 µM or less, preferably 100 µM or less, more preferably 10 µM or less. In one embodiment, the concentration of SAG in the medium is generally 1 nM to 1000 µM, preferably 10 nM to 100 µM, more preferably 100 nM to 10 µM (e.g., 2 µM).

In a preferred embodiment, the medium used in the third culture step contains FGF2. FGF2 promotes differentiation of pituitary placode and/or Rathke's pouch into pituitary hormone-producing cells.

The definition of FGF2 is as described in the second culture step.

The concentration of FGF2 in the medium is not particularly limited as long as it can promote differentiation of pituitary placode and/or Rathke's pouch into pituitary hormone-producing cells. It is generally 1 ng/ml or more, preferably 10 ng/ml or more. The upper limit of the FGF2 concentration is not particularly set as long as differentiation into pituitary hormone-producing cells is not adversely affected. From the aspect of culture cost, it is generally 1000 ng/ml or less, preferably 500 ng/ml or less. In one embodiment, the concentration of FGF2 in the medium is generally 1 to 1000 ng/ml, preferably 10 to 100 ng/ml.

In a preferred embodiment, the medium used in the third culture step contains a Notch signal inhibitor. A Notch signal inhibitor promotes differentiation of pituitary placode and/or Rathke's pouch into pituitary hormone-producing cells (particularly, ACTH-producing cells). The Notch signal inhibitor increases the expression of transcription factor Tbxl9 that upregulates ACTH production.

The Notch signal inhibitor is not particularly limited as long as it can suppress signal transduction mediated by Notch. Examples of the Notch signal inhibitor include gamma secretase inhibitors such as DAPT (N-[N-(3,5-difluorophenacetyl)-1-alanyl]-S-phenylglycine t-butyl ester), DBZ, MDL28170, and the like. Among these, DAPT is preferred.

The concentration of the Notch signal inhibitor in the medium is not particularly limited as long as it can promote differentiation of pituitary placode and/or Rathke's pouch into pituitary hormone-producing cells (particularly, ACTH-producing cells). For example, in the case of DAPT, it is generally 0.1 µM or more, preferably 1 µM or more. The upper limit of the DAPT concentration is not particularly set as long as differentiation into pituitary hormone-producing cells is not adversely affected. From the aspect of culture cost, it is generally 1000 µM or less, preferably, 100 µM or less. In one embodiment, the concentration of DAPT in the medium is generally 0.1 to 1000 µM, preferably 1 to 100 µM (e.g., 10 µM).

In the third culture step, addition of a bone morphogenic factor signal transduction pathway activation substance to the medium is not necessary. In one embodiment, the medium used in the third culture step does not contain a bone morphogenic factor signal transduction pathway activation substance.

FGF2 used in the present invention is preferably isolated. The isolated FGF2 contained in the medium used in the third culture step is exogenously added to the medium. Therefore, in one embodiment, the present invention includes a step of exogenously adding an isolated FGF2 to the medium used in the second culture step.

In the third culture step, the cell aggregates may be treated with corticosteroids by adding corticosteroids to the medium. The treatment with corticosteroids promotes differentiation of pituitary placode and/or Rathke's pouch into pituitary hormone-producing cells other than ACTH-producing cells (i.e., GH-producing cells, PRL-producing cells, TSH-producing cells, LH-producing cells, FSH-producing cells, etc.). Examples of the corticosteroids include, but are not limited to, natural glucocorticoids such as hydrocortisone, cortisone acetate, acetic acid fludrocortisone, and the like; artificially-synthesized glucocorticoids such as dexamethasone, betamethasone, predonisolone, methylprednisolone, triamcinolone, and the like, and the like.

The concentration of corticosteroids in the medium is not particularly limited as long as it can promote differentiation of pituitary placode and/or Rathke's pouch into pituitary hormone-producing cells (excluding ACTH-producing cells). It can be appropriately determined according to the kind of the corticosteroids. For example, in the case of hydrocortisone, it is generally 100 ng/ml or more, preferably 1 µg/ml or more. The upper limit of the hydrocortisone concentration is not particularly set as long as differentiation into pituitary hormone-producing cells (excluding ACTH-producing cells) is not adversely affected. From the aspect of culture cost, it is generally 1000 µg/ml or less, preferably 100 µg/ml or less. In one embodiment, the concentration of hydrocortisone in the medium is generally 100 ng/ml to 1000 µg/ml, preferably 1 to 100 µg/ml. When dexamethasone is used as the corticosteroids, the concentration thereof in the medium can be set to about 1/25 that of hydrocortisone.

In the third culture step, the timing of adding corticosteroids to the medium is not particularly limited as long as it can promote differentiation of pituitary placode and/or Rathke's pouch into pituitary hormone-producing cells (excluding ACTH-producing cells). Corticosteroids may be added to the medium from the start of the third culture step, or corticosteroids may be added to the medium after culturing for a certain period of time in a medium free of addition of corticosteroids after the start of the third culture step. Preferably, after the start of the third culturing step, corticosteroids are added to the medium when the emergence of ACTH-producing cells is confirmed in the cell aggregates. That is, the cell aggregates are cultured in a medium free of addition of corticosteroids until the emergence of ACTH-producing cells is confirmed in the cell aggregates, and the third culture step is continued in a medium containing corticosteroids, after the emergence of ACTH-producing cells is confirmed. The emergence of ACTH-producing cells can be confirmed by immunohistological staining using an antibody against ACTH. When human pluripotent stem cells are used, the emergence of ACTH-producing cells can be generally expected after 37 days from the start of the third culture step. In one embodiment, therefore, corticosteroids are added to the medium 37 days after the start of the third culture step.

The period of treatment of cell aggregates with corticosteroids is not particularly limited as long as it can promote differentiation of pituitary placode and/or Rathke's pouch into pituitary hormone-producing cells (excluding ACTH-producing cells). In general, cell aggregates are treated with corticosteroids until promotion of differentiation into pituitary hormone-producing cells (excluding ACTH-producing cells) is confirmed in the corticosteroid treatment group as compared with a corticosteroid non-treatment group. The treatment period is generally 7 days or more, preferably 12 days or more. The upper limit of the treatment period is not particularly set and the corticosteroids may be removed from the medium at the stage when promotion of differentiation into pituitary hormone-producing cells (excluding ACTH-producing cells) is confirmed in the corticosteroid treatment group as compared with the corticosteroid non-treatment group.

The addition of corticosteroids to the medium suppresses differentiation induction of ACTH-producing cells by feedback inhibition.

The medium used in the third culture step can be prepared using a medium used for culturing mammalian cells as a basal medium, similar to the medium used in the first and the second culture step. Examples of the basal medium include BME medium, BGJb medium, CMRL 1066 medium, Glasgow MEM medium, Improved MEM Zinc Option medium, IMDM medium, medium 199 medium, Eagle MEM medium, αMEM medium, DMEM medium, Ham medium, Ham's F-12 medium, RPMI1640 medium, Fischer's medium, Neurobasal medium and a mixed medium of these (e.g., DMEM/F-12 medium (mixed medium of DMEM medium:Ham's F-12 medium-1:1) etc.) and the like, and the medium is not particularly limited as long as it can be used for culturing mammalian cells. In one embodiment, a mixed medium of IMDM medium and Ham's F-12 medium is used. The mixing ratio is, for example, IMDM:Ham's F-12=0.8 to 1.2:1.2 to 0.8 in volume ratio.

The medium used for culture may be a serum-containing medium or serum-free medium. To avoid contamination with chemically undetermined components, the medium to be used for the suspension culture of cell aggregates is preferably a serum-free medium.

The medium used for suspension culture of cell aggregates may contain a serum replacement. The serum replacement may contain, for example, albumin, transferrin, fatty acid, collagen precursor, trace element, 2-mercaptoethanol or 3'thiolglycerol, or an equivalent of these and the like as appropriate. Such serum replacement can be prepared, for example, by the method described in WO 98/30679. To perform the method of the present invention more simply, a commercially available serum replacement can be utilized. Examples of such commercially available serum replacement include KSR (knockout serum replacement) (manufactured by Invitrogen), Chemically-defined Lipid concentrated (manufactured by Gibco), and Glutamax (manufactured by Gibco).

The medium used for suspension culture of cell aggregates may contain other additives as long as it does not adversely affect induction of differentiation of pluripotent stem cells into pituitary placode and/or Rathke's pouch, and then pituitary hormone-producing cells. Examples of the additive include, but are not limited to, insulin, iron source (e.g., transferrin, etc.), mineral (e.g., sodium selenite, etc.), saccharides (e.g., glucose, etc.), organic acid (e.g., pyruvic acid, lactic acid, etc.), serum protein (e.g., albumin, etc.), amino acid (e.g., L-glutamine, etc.), reducing agent (e.g., 2-mercaptoethanol, etc.), vitamins (e.g., ascorbic acid, d-biotin, etc.), antibiotic (e.g., streptomycin, penicillin, gentamicin, etc.), buffering agent (e.g., HEPES, etc.) and the like.

In one embodiment, from the viewpoint of not adversely affecting the induction of differentiation into pituitary hormone-producing cells, the medium used for suspension culture of cell aggregates is a chemically defined medium that does not contain growth factors other than those particularly described in the present specification to be contained in the medium (growth-factor-free Chemically Defined medium; gfCDM) and is supplemented with a serum replacement (KSR etc.). The "growth factor" here includes Fgf; BMP; pattern formation factors such as Wnt, Nodal, Notch, Shh, and the like; and insulin and Lipid-rich albumin. As the chemically defined medium not containing a growth factor, for example, gfCDM disclosed in Wataya et al, Proc Natl Acad Sci USA, 105(33): 11796-11801, 2008 can be mentioned.

The suspension culture in the third culture step is preferably performed under high oxygen partial pressure conditions. Further suspension culture of cell aggregates containing 1) hypothalamic neuroepithelial tissue, and 2) pituitary placode and/or Rathke's pouch under high oxygen partial pressure conditions allows oxygen to reach the inside of the cell aggregates and achieves maintenance culture of the cell aggregates for a long period of time, which in turn enables efficient induction of differentiation into pituitary hormone-producing cells.

The high oxygen partial pressure conditions mean oxygen partial pressure conditions above the oxygen partial pressure in air (20%). The oxygen partial pressure in the third culture step is, for example, 30 to 60%, preferably 35 to 60%, more preferably 38 to 60%.

Other culture conditions such as culture temperature, CO₂ concentration, and the like in the third culture step can be appropriately set. The culture temperature is, for example, about 30 - 40°C, preferably about 37°C. The CO₂ concentration is, for example, about 1 to 10%, preferably about 5%.

The third culture step is conducted for a period of time sufficient to induce differentiation of pituitary placode and/or Rathke's pouch into pituitary hormone-producing cells. By performing the third culture step, differentiation of pituitary placode and/or Rathke's pouch into pituitary hormone-producing cells is induced, pituitary hormone-producing cells are produced in the pituitary placode and/or Rathke's pouch, and adenohypophysis can be formed. As the pituitary hormone-producing cells induced from pituitary placode and/or Rathke's pouch, growth hormone (GH)-producing cells, prolactin (PRL)-producing cells, and adrenocorticotropic hormone (ACTH)-producing cells can be mentioned. In a preferred embodiment, the adrenocorticotropic hormone (ACTH)-producing cells secrete ACTH in response to CRH stimulation, and the ACTH secretion is feedback-suppressed by glucocorticoid. In one embodiment, differentiation of at least one type, preferably two types, more preferably three types, of pituitary hormone-producing cells selected from the group consisting of growth hormone (GH)-producing cells, prolactin (PRL)-producing cells, and adrenocorticotropic hormone (ACTH)-producing cells from pituitary placode and/or Rathke's pouch is induced, and adenohypophysis containing one type, preferably two types, more preferably three types, of pituitary hormone-producing cells selected from the group consisting of growth hormone (GH)-producing cells, prolactin (PRL)-producing cells, and adrenocorticotropic hormone (ACTH)-producing cells is formed. From pituitary placode and/or Rathke's pouch, other pituitary hormone-producing cells such as thyroid-stimulating hormone (TSH)-producing cells, follicle-stimulating hormone (FSH)-producing cells, luteinizing hormone (LH)-producing cells, melanocyte stimulation hormone (MSH)-producing cells and the like may be induced besides growth hormone (GH)-producing cells, prolactin (PRL)-producing cells, and adrenocorticotropic hormone (ACTH)-producing cells. That is, the adenohypophysis formed by the third culture step may contain other pituitary hormone-producing cells such as thyroid-stimulating hormone (TSH)-producing cells, follicle-stimulating hormone (FSH)-producing cells, luteinizing hormone (LH)-producing cells, melanocyte stimulation hormone (MSH)-producing cells and the like in addition to at least one type, preferably two types, more preferably three types, selected from the group consisting of growth hormone (GH)-producing cells, prolactin (PRL)-producing cells, and adrenocorticotropic hormone (ACTH)-producing cells. Differentiation into pituitary hormone-producing cells can be confirmed, for example, by detecting the pituitary hormone positive cell by immunohistochemistry using specific antibodies against pituitary hormone. For example, the third culture step is conducted until 10% or more, preferably 30% or more, more preferably 50% or more, of the cell aggregates in the culture contain pituitary hormone-producing cells. The culture period may vary according to the animal species of the pluripotent stem cells, and the kind of the Shh signal pathway agonist and cannot be specified. When a human pluripotent stem cell is used, the period of the third culture step is, for example, generally not less than 37 days, for example, 37 to 70 days.

By performing the third culture step, cell aggregates containing adenohypophysis can be obtained.

Through the production method of the present invention, suspension culture of aggregates may be performed in the presence or absence of feeder cells as long as differentiation of pluripotent stem cells into adenohypophysis or a precursor tissue thereof can be induced. However, from the viewpoint of avoiding contamination with undetermined factors, it is preferable to perform suspension culture of cell aggregates in the absence of feeder cells.

In the production method of the present invention, a culture vessel used for the suspension culture of cell aggregates is not particularly limited and, for example, flask, tissue culture flask, dish, petri dish, tissue culture dish, multidish, microplate, micropore, microwell plate, multiplate, multiwall plate, chamber slide, petri dish, tube, tray, culture bag, and roller bottle can be mentioned. To enable culture under non-adhesive conditions, the culture vessel is preferably non-adhesive to cells. As the cell non-adhesive culture vessel, a culture vessel whose surface has been artificially treated to be non-adhesive to cells, a culture vessel that has not been artificially treated (for example, coating treatment with extracellular matrix, etc.) for the purpose of improving adhesion to cells, or the like can be used.

As the culture vessel to be used for the suspension culture of cell aggregates, an oxygen-permeable culture vessel may also be used. By using an oxygen-permeable culture vessel, the supply of oxygen to cell aggregates is improved, which can contribute to the long-term maintenance culture of cell aggregates.

In suspension culture of aggregates, the aggregates may be statically cultured or the aggregates may be intentionally moved by rotation culture or shaking culture, as long as the aggregates can maintain a non-adhesive state to the culture vessel. In the present invention, it is not necessary to intentionally move aggregates by rotation culture or shaking culture. That is, in one embodiment, the suspension culture in the production method of the present invention is performed by static culture. The static culture refers to a method of culturing aggregates without intentionally moving them. That is, for example, the medium is circulated as the topical medium temperature changes, and the aggregates may move due to the flow, even though the aggregates are not intentionally moved. In the present invention, such case is also included in the static culture. Static culture may be performed throughout the entire suspension culture period, or static culture may be performed only for a part of the period. In a preferred embodiment, static culture is performed throughout the entire suspension culture period. Static culture does not require equipment, is expected to cause less damage to cell aggregate, and is advantageous in that the volume of culture medium can be reduced.

### (4) Separation method of pituitary hormone-producing cells and the like using cell surface marker of the present invention

The present invention provides a method for separating pituitary hormone-producing cells and/or progenitor cells thereof, including a step of separating the cells that express EpCAM from cell aggregates containing adenohypophysis and/or a precursor tissue thereof. EpCAM may be expressed on the surface of pituitary hormone-producing cells and progenitor cells thereof, whereas EpCAM cannot be expressed on the surface of hypothalamic neuroepithelial tissue. Therefore, pituitary hormone-producing cells and progenitor cells thereof, and hypothalamic neuroepithelial tissue can be separated using EpCAM as a marker.

The cell aggregate containing adenohypophysis and/or a precursor tissue thereof to be used in the separation method of the present invention may be any of the cell aggregate obtained by the second culture step, and the cell aggregate obtained by the third culture step. A specific number of days after the start of differentiation induction from the aforementioned pluripotent stem cells into pituitary gland or a partial tissue thereof, or a precursor tissue thereof is, for example, 30 days to 600 days, preferably 90 days to 500 days, more preferably 150 days to 400 days, further preferably 200 days to 350 days. The separation method of the present invention includes the following steps.

### Outline of separation steps of the present invention

The method of the present invention for separating pituitary hormone-producing cells and/or progenitor cells thereof from cell aggregates containing adenohypophysis and/or a precursor tissue thereof, including a step of separating the cells that express EpCAM may include (A) a step of dispersing cell aggregates containing adenohypophysis and/or a precursor tissue thereof to obtain a population of single cells, before (B) a step of separating cells (cell population) that expresses EpCAM.

The step (A) of dispersing cell aggregates to obtain a population of single cells may specifically include (al) a step of dispersing cell aggregates containing adenohypophysis and/or a precursor tissue thereof by an enzyme treatment (below-mentioned (4.2)). Before the step of dispersing by an enzyme treatment, (a2) a step of shredding the cell aggregates containing adenohypophysis and/or a precursor tissue thereof, or physically incising the cell aggregates containing adenohypophysis and/or a precursor tissue thereof may be included (below-mentioned (4.2)).

The step (A) of dispersing cell aggregates to obtain a population of single cells may optionally include (a-1) a step of treating cell aggregates containing adenohypophysis and/or a precursor tissue thereof with a ROCK inhibitor. When this step is performed, it is preferably performed first in the step of obtaining a population of single cells (below-mentioned (4.1)).

After the step (a2) of dispersing by enzymatic treatment, (B) a step of separating cells (cell population) expressing EpCAM (step of separating cells (cell population) expressing EpCAM and cells (cell population) not expressing EpCAM) is performed (below-mentioned (4.3)).

After the above-mentioned cell (cell population) separation step (B), a step (C) of reaggregating the obtained cells (cell population) may be optionally performed (below-mentioned (4.4)). The reaggregation step (C) may be specifically performed by (cl) seeding the obtained cells (cell population) in a culture vessel and performing adhesion culture, and/or (c2) seeding the obtained cells (cell population) in a culture vessel and performing suspension culture.

### (4.1) Pre-treatment step with ROCK inhibitor

In the separation method of the present invention, the obtained cell aggregates may be first pre-treated with a ROCK inhibitor. For the pre-treatment, a ROCK inhibitor is preferably added before the start of the pre-treatment in order to suppress cell death of pluripotent stem cells (particularly, human pluripotent stem cells) induced by dispersing of the cell aggregate thereafter. The ROCK inhibitor is added, for example, at least 24 hr before, at least 12 hr before, at least 6 hr before, at least 3 hr before, at least 2 hr before, at least 1 hr before, the start of the treatment. As the ROCK inhibitor, Y-27632((+)-(R)-trans-4-(1-aminoethyl)-N-(4-pyridyl)cyclohexanecarboxamide dihydrochloride) and the like can be mentioned. The concentration of the ROCK inhibitor used for the treatment is a concentration that can suppress cell death of pluripotent stem cells induced by dispersing of the cell aggregate thereafter. For example, for Y-27632, such concentration is generally about 0.1 to 200 µM, preferably about 2 to 50 µM. The concentration of the ROCK inhibitor may be varied during the period of addition, and, for example, the concentration can be halved in the latter half of the period. In any of the below-mentioned steps (4.2) to (4.4), as the solution to be contacted with the cells, a solution containing a ROCK inhibitor at the same concentration is preferably used.

As the medium to perform the pre-treatment, the medium used in the second culture step can be used when the cell aggregates obtained in the second culture step is used, and the medium used in the third culture step can be used when the cell aggregates obtained in the third culture step is used. When a ROCK inhibitor is already added to the above-mentioned medium at a desired concentration, the pre-treatment step is not necessary.

### (4.2) Cell aggregate dispersion step

Then, the cell aggregates that underwent the above-mentioned pre-treatment are dispersed by an enzyme treatment. Specifically, the cell aggregates that underwent the above-mentioned pre-treatment are transferred to a culture vessel (e.g., tube etc.) containing the medium (e.g., mixed medium of DMEM/F-12 medium (DMEM medium:Ham's F-12 medium=1:1), etc.) described in the second or the third culture step and washed with the same medium. The enzyme for dispersion is not particularly limited as long as it can disperse the cells. For example, EDTA; enzymes such as trypsin, collagenase (collagenase type I - VII), metalloprotease, hyaluronidase, elastase, DISPASE, deoxyribonuclease, and the like, and a mixture of these can be mentioned. A preferred enzyme is collagenase, and more preferred enzyme is collagenase type I. The conditions (temperature, time, etc.) of the enzyme treatment can be appropriately set according to the enzyme to be used and the like. To promote the enzyme treatment, a step of physically (e.g., scalpel, scissors, etc.) shredding the cell aggregate, or physically (e.g., scalpel, scissors, etc.) incising the cell aggregates may be performed before the treatment.

After the above-mentioned enzyme treatment, floating cells are recovered, and the above-mentioned enzyme treatment is performed again. The enzyme treatment can be performed using the aforementioned enzyme and the like, and preferred enzymes are EDTA; trypsin, more preferably, EDTA; trypsin and deoxyribonuclease. In addition, a commercially available product such as TrypLE (Invitrogen) or the like may also be used instead of EDTA; trypsin. The conditions (temperature, time, etc.) of the enzyme treatment can be appropriately set according to the enzyme to be used and the like. A single cell suspension can be prepared by the above-mentioned series of enzyme treatments. When preparing a single cell suspension, dead cells may be removed by a method known per se.

### (4.3) Separation step of EpCAM-positive cell

As a method for separating the desired cells that express EpCAM from the above-mentioned cell population contained in the single cell suspension prepared above, methods using flow cytometry and mass cytometry, magnetic cell separation methods, and the like can be mentioned. These methods can be carried out using a method known per se. For example, cell that expresses EpCAM can be separated by a method including a step of contacting the cells and a substance (e.g., antibody, etc.) that specifically binds to EpCAM molecule. The above-mentioned substance includes those with detectable labels (e.g., GFP, PE) attached to themselves and those without labels attached to themselves. When the above-mentioned substance does not have a label attached thereto, the separation can be achieved by further using a substance with a detectable label attached thereto that directly or indirectly recognizes the substance. For example, when the aforementioned substance is an antibody, then a fluorescent dye, a metal isotope, or a bead (e.g., magnetic bead) is directly or indirectly carried on the antibody to label a cell surface marker. The cells can be separated based on the label. The antibody used at this time may be one kind of antibody, or two or more kinds of antibodies.

### (4.4) Reaggregation culture step of separated cell population

The separated cell population may be seeded in a culture vessel and subjected to adhesion culture to give, for example, a cell sheet, or may be seeded in a culture vessel and subjected to suspension culture to allow for reaggregation to form a cell aggregate. The cell population may be maintenance cultured as it is until use, or may be further induced to differentiate into desired cells according to the differentiation state of the separated cells. When the maintenance culture or further differentiation induction is performed, the method described in the aforementioned third culture step may be performed as it is, or where necessary, it may be performed by appropriately modifying by a method known per se. When the adhesion culture is performed, the adhesion culture itself can be performed by a method known per se, and the contents described in the aforementioned third culture step (e.g., medium composition, etc.) may also be used as appropriate. During the adhesion culture, it is preferable to coat the culture vessel with an extracellular matrix or the like (e.g., laminin, collagen, etc.).

### (5) Use of separated pituitary hormone-producing cells and progenitor cells thereof

The pituitary hormone-producing cells and progenitor cells thereof obtained by the separation method or the production method of the present invention can be used for transplantation therapy. For example, the pituitary hormone-producing cells and progenitor cells thereof obtained by the separation method or the production method of the present invention can be used as a therapeutic drug for diseases based on disorders of the adenohypophysis (anterior or intermediate lobe, preferably anterior lobe), or for supplementing the corresponding damaged portion in the damaged states of the adenohypophysis (anterior or intermediate lobe, preferably the anterior lobe). By transplanting the pituitary hormone-producing cells and progenitor cells thereof obtained by the separation method or the production method of the present invention to patients with diseases based on disorders of the adenohypophysis or in the damaged states of the adenohypophysis, the diseases based on disorders of the adenohypophysis and the damaged states of the adenohypophysis can be treated. The transplantation site is not particularly limited as long as the transplanted pituitary hormone-producing cells and/or progenitor cells thereof can function as a substitute for the damaged adenohypophysis and, for example, under the renal capsule and the like can be mentioned. As the diseases based on disorders of the adenohypophysis, panhypopituitarism, pituitary dwarfism, adrenocortical insufficiency, partial hypopituitarism, pituitary anterior hormone isolated deficiency, and the like can be mentioned. As the damaged states of the adenohypophysis, patients after adenohypophysectomy, patients after radiation to pituitary tumors, and trauma can be mentioned.

In transplantation therapy, rejection due to differences in histocompatibility antigens often poses a problem. Such problem can be overcome by using pluripotent stem cells (e.g., iPS cells) established from the somatic cells of recipients of transplantation. That is, in a preferred embodiment, pluripotent stem cells (e.g., iPS cells) established from the somatic cells of recipients are used as pluripotent stem cells in the method of the present invention to produce adenohypophysis or a precursor tissue thereof, or a pituitary hormone-producing cell, which is immunologically self for the recipient, and this is transplanted into the recipient.

Furthermore, the pituitary hormone-producing cells and progenitor cells thereof obtained by the method of the present invention can be used for screening and evaluation of drugs. Specifically, they can be applied to, for example, screening for substances that suppress or promote the production of pituitary hormones, side effects and toxicity tests of pharmaceutical products, and the like. The above-mentioned screening, test, and the like may contain, for example, a step of culturing a cell population containing pituitary hormone-producing cells and/or progenitor cells thereof in the presence or absence (negative control) of a test substance, a step of comparing the production amount of the desired hormone in a cell population treated with a test substance with that of a negative control, and a step of selecting, as a candidate substance, a test substance that suppresses or promotes the production of the pituitary hormone.

### (5) Production method of pituitary hormone-producing cells and/or progenitor cells thereof of the present invention

The present invention provides a method for producing pituitary hormone-producing cells and/or progenitor cells thereof, including a step of separating the cells that express EpCAM from cell aggregates containing adenohypophysis and/or a precursor tissue thereof. In the present invention, the pituitary hormone-producing cells and/or progenitor cells thereof may be pituitary hormone-producing cells and/or progenitor cells thereof, or a population (cell population) of pituitary hormone-producing cells and/or a population (cell population) of progenitor cells thereof, or a cell population containing pituitary hormone-producing cells and/or progenitor cells thereof in a high purity. EpCAM may be expressed on the surface of pituitary hormone-producing cells and progenitor cells thereof, whereas EpCAM cannot be expressed on the surface of hypothalamic neuroepithelial tissue. Therefore, pituitary hormone-producing cells and progenitor cells thereof, and hypothalamic neuroepithelial tissue can be separated using EpCAM as a marker. Using the separation step, a cell population containing pituitary hormone-producing cells and/or progenitor cells thereof, or pituitary hormone-producing cells and/or progenitor cells thereof in a high purity (e.g., 80% or more, preferably 85% or more, more preferably 90%, further preferably 95% or more, further more preferably 99% or more, most preferably 100% of the cell population) can be produced.

In the step of separating the cells that express EpCAM from cell aggregates containing adenohypophysis and/or a precursor tissue thereof contained in the production method of the present invention, all of the contents described in the aforementioned "Outline of separation step of the present invention" may be used. In addition, all of the contents of the aforementioned (4.1) to (4.4) can be used for each step.

The pituitary hormone-producing cells (population) and/or progenitor cells thereof (population), and the like produced by the method of the present invention may be, as described in the above-mentioned (4.1), maintenance cultured as they are until use, or may be further induced to differentiate into desired cells according to the differentiation state of the separated cells, by appropriately culturing the cells (e.g., the third culture step). Specifically, for example, the pituitary hormone-producing cells (population) and/or progenitor cells thereof (population) and the like produced by the method of the present invention may be directly transplanted to a subject in need of the treatment and the like with the cell population, or may be further processed by a method known per se to achieve higher purity (purified) and then transplanted to the subject. The purified cells (population) may be seeded in a culture vessel and adhesion cultured as described in the above-mentioned (4.4) and, for example, may be shaped like a cell sheet, or seeded in a culture vessel and cultured in suspension to cause reaggregation. A cell sheet or cell aggregate obtained through the adhesion culture or suspension culture may be transplanted to the above-mentioned subject. The adhesion culture or suspension culture may be performed for a short period of time (e.g., 3 to 6 days) or for a long period of time (e.g., 7 to 30 days). In the production method of the present invention, all of the contents relating to the aforementioned "(1) Pluripotent stem cell" to "(5) Use of separated pituitary hormone-producing cells and progenitor cells thereof" may be used.

While the present invention is explained in more detail in the following by referring to Examples, the Examples show mere exemplification of the present invention and do not limit the scope of the present invention in any manner.

### [Example]

### Example 1: Screening for cell surface antigen

Human iPS cells (201B7 strain) were induced to differentiate into cell aggregates containing adenohypophysis or a precursor tissue thereof, by the method known per se and described in WO 2016/013669 or Cell Reports, 30, 18-24, January 7, 2020.

The cell aggregates on day 47 from the differentiation induction of the human iPS cells (201B7 strain) were dispersed by treating with Accumax (Innovative Cell Technologies, #AM105) at 37°C for 5 to 10 min to prepare a single cell suspension. The cells were stained with human surface antigen PE-labeled antibody panel (LEGENDScreen Human PE Kit, #700007) manufactured by BioLegend, and subjected to fixation and permeabilization treatment using IntraStain (Dako, #K2311). Successively, intracellular Cytokeratin staining with FITC-labeled anti-Cytokeratin antibody (Miltenyi, #130-080-101), nuclear staining with Hoechst 33342, and cytoplasm staining with HCS CellMask Deep Red Stain (Invitrogen, #H32721) were performed. The images of the cells after staining were obtained and analyzed by an imaging analyzer (Opera Phenix) manufactured by PerkinElmer, and surface antigens with a high coexistence rate in cytokeratin-positive cells were searched for. As a result, it was suggested that EpCAM can identify pituitary progenitor cells.

### Example 2: Quantification of expression rate of EpCAM and Cytokeratin

In the same manner as in screening for cell surface antigens, a single cell suspension was prepared, and staining was performed using PE-labeled anti-EpCAM antibody (Miltenyi, #130-098-115), FITC-labeled anti-Cytokeratin antibody, Hoechst 33342, and images were obtained with a fluorescence microscope (Leica DMI6000B). Using a Cell counter tool of image analysis software Image J, EpCAM+/Cytokeratin+ cells, EpCAM+/Cytokeratin- cells, EpCAM-/Cytokeratin+ cells, and EpCAM-/Cytokeratin- cells were counted, and the ratio of each to the total cells was calculated. As a result, as a surface antigen highly specific to cytokeratin-positive cells, an epithelial cell adhesion molecule (EpCAM; also known as CD326) was identified (Fig. 1 A). The EpCAM was expressed in about 80% of Cytokeratin-positive cells, and about 95% of EpCAM-positive cells were Cytokeratin-positive (Fig. 1 B).

### Example 3: Purification of EpCAM-positive cell by magnetic cell separation method (Magnetic-Activated Cell Sorting; MACS)

Cell aggregates 90 to 500 days after initiation of differentiation induction were enzymatically dispersed and used for MACS. The procedure is shown below.

### (1) Pre-treatment with Y27632

In order to suppress cell death due to dispersion treatment, Y27632 (Wako, #034-24024) was added to the medium at a final concentration of 20 µM one hour or more before the start of the work to pre-treat cell aggregates. In subsequent operations, 20 µM Y27632 was added to all solutions (except PBS) to which the cells were exposed.

### (2) Dispersion of cell aggregates

In order to promote dispersion by enzyme treatment, the cell aggregates were shredded or incised with a scalpel. Cell aggregates were then transferred to a 50 ml tube and washed with DMEM/F12 (Wako, #042-30555). Then 1-3 ml of collagenase solution was added and the mixture was rotated or shaken (140-150 rpm) at 37°C for 40 min. The composition of the collagenase solution was the above-mentioned DMEM/F12 supplemented with 0.2% collagenase type I (Wako, #031-17601) and 0.1% BSA (Sigma, #A9418).

After the completion of collagenase treatment, the supernatant containing floating cells was transferred to a new 15 ml tube, and the remaining cell aggregates were washed with PBS (washing liquid was also collected in the same tube as the supernatant). The 15 ml tube in which the supernatant was collected was centrifuged at 4°C, 1000 rpm for 5 min to pelletize floating cells. 1 ml of 0.25% Trypsin/EDTA (Gibco, #25200072) + 0.2 mg/ml DNase I (Roche, #11284932001) was added to the cell aggregates in the 50 ml tube, incubated at 37°C for 5-10 min, combined with the pellets in the 50 ml tube, and the cell aggregates were loosened by pipetting about 20 times with a P1000 micropipette. After suspending in 10 ml of gfCDM + 20% KSR (medium for differentiation and maintenance of cell aggregates) to neutralize trypsin, the cells were centrifuged at 4°C, 1000 rpm for 5 min. 1 ml of gfCDM + 20% KSR + 10 µg/ml DNase I was added to the cell pellets after centrifugation, vigorously pipetted about 30 times with a P1000 micropipette to disperse the cell aggregates, and aggregates were removed through a 70 µm cell strainer. A single cell suspension was prepared by the following operation.

### (3) Removal of dead cells

The dispersed cells were treated with a dead cell removal kit (Miltenyi, #130-090-101) to remove dead cells and apoptotic cells.

### (4) Separation of EpCAM-positive cells by MACS

The collected viable cells were suspended in MACS buffer (PBS + 0.5% BSA + 2 mM EDTA) and stained with PE-labeled anti-EpCAM antibody (refrigerated for 10 min). EpCAM-positive cells were successively labeled with magnetic beads by treating with anti-PE-microbeads (Miltenyi, #130-105-639). Then the cells were passed through a magnetic column (LS column; Miltenyi, #130-042-401) to separate and collect magnetic bead-labeled cells (EpCAM-positive cells) and unlabeled cells (EpCAM-negative cells).

### Example 4: Reaggregation culture of purified cells

Each purified cell population (EpCAM-positive cells, negative cells) was seeded on a low-adhesion V-bottom 96-well plate (PrimeSurface plate 96V; Sumitomo Bakelite Co., Ltd., #MS-9096V) and allowed to reaggregate. 15,000 cells in 200 µl medium (gfCDM + 20% KSR + 30 µM Y27632) were seeded per well. Thereafter, half of the medium was replaced every 3 days with a medium not containing Y27632.

### Example 5: Immunohistochemistry

Cell aggregates cultured for 48 days or more after the start of differentiation induction and cell aggregates reaggregated after MACS purification and cultured for 6 days or more were used. The cell aggregates were fixed with 4% paraformaldehyde solution, and sucrose replacement was performed by stepwise immersion in 10, 20, and 30% sucrose solutions. Successively, the cell aggregates were embedded with an OCT compound, and cryosections with a thickness of 4 to 10 µm were produced with a cryostat and attached to anti-peeling coated slide glass (PLATINUM PRO; Matsunami Glass). The sections were washed with PBS, and blocked with a blocking solution (5% normal donkey serum + 0.1% TritonX-100 in PBS) for 30 to 60 min at room temperature. Then, they were reacted overnight at 4°C with a primary antibody diluted with a blocking solution. Successively, they were reacted at room temperature for 1 hr with a fluorescence-labeled secondary antibody and DAPI (nuclear staining agent). The sections after staining were mounted with Fluoromount (Diagnostic BioSystems, #K024) and subjected to fluorescence observation with a confocal laser microscope LSM-710 (Zeiss).

As a result, EpCAM-positive thickened epithelial tissue was observed on the surface of the aggregates. This EpCAM-positive epithelial tissue was 3 cells or more thick in the vertical direction. It was found that this EpCAM-positive was co-positive with Cytokeratin (Fig. 2 A). Furthermore, this EpCAM-positive was found to be co-positive with Pitx1 (Fig. 2 B) .

### Example 6: ACTH secretion test (CRH stimulation test)

Cell aggregates that were reaggregated after MACS purification and cultured for 6 days or more were used. Six cell aggregates were transferred to a 1.5 ml microtube and incubated in 250 µl of HBSS(+) (Wako, # 084-08965) at 37°C for 10 min, and the HBSS was recovered. Successively, the cells were incubated in 250 µl of HBSS containing 1 ug/ml CRH (Peptide Institute, #4136-s) at 37°C for 10 min, and the HBSS was recovered. The ACTH concentration in the recovered HBSS was measured by the ECLIA method used in clinical testing (testing commissioned to SRL Co., Ltd.).

### Results

Adenohypophysis and a precursor tissue thereof are expressed using Cytokeratin as a marker. Cytokeratin is a cytoskeletal protein and can be detected in a short time and with high sensitivity by immunostaining. Therefore, cell aggregates induced to differentiate from human iPS cells were dispersed, and surface antigen screening was performed using a commercially available antibody panel and targeting cytokeratin-positive cells. As the time of differentiation for screening, around 50 days of culture was selected when pituitary progenitor cells have already been induced and tissue can be dispersed by a gentle enzyme treatment. As a result, the degradation of surface antigens could be prevented and a sufficient amount of cells for screening could be secured. As a result of screening of 332 kinds of human surface antigen, as a surface antigen highly specific to cytokeratin-positive cells, an epithelial cell adhesion molecule (EpCAM; also known as CD326) was identified (Fig. 1 A). The EpCAM was expressed in about 80% of Cytokeratin-positive cells, and about 95% of EpCAM-positive cells were Cytokeratin-positive (Fig. 1 B).

Immunohistochemical analysis of cell aggregates revealed that EpCAM was expressed in Cytokeratin-, Pitx1-, or Lhx3-positive pituitary progenitor cells (Figs. 2 A, B, C). In addition, EpCAM expression was also maintained in pituitary hormone-producing cells (ACTH-positive cells) differentiated therefrom (Fig. 2 C).

Using cell aggregates that were induced to differentiate for 90 days or more, EpCAM-positive and EpCAM-negative cells were separated by MACS to attempt reaggregation (Fig. 3 A). Both EpCAM-positive and EpCAM-negative cell populations formed aggregates. It was confirmed that EpCAM-positive cell aggregates contained ACTH-positive cells and Lhx3-positive cells, whereas EpCAM-negative cell aggregates did not contain ACTH-positive cells and Lhx3-positive cells (Fig. 3 B). As a result of an ACTH secretion test, in the EpCAM-positive cell aggregates, the ACTH secretion amount increased to 2.1-fold on average by the addition of CRH, a physiological secretion stimulation factor (Fig. 3 C). On the other hand, EpCAM-negative cell aggregates did not show an increase in ACTH secretion by the addition of CRH, and the secretion amount before CRH addition was less than half that of EpCAM-positive cell aggregates on average (Fig. 3 C).

From the above results, it is understood that functional pituitary hormone-producing cells and progenitor cells thereof can be purified from differentiated tissues derived from human pluripotent stem cells by using EpCAM as a marker.

### [Industrial Applicability]

The separation method and the production method of the present invention can be used to efficiently separate and purify functional pituitary hormone-producing cells and/or progenitor cells thereof from differentiated tissues derived from human pluripotent stem cells, by utilizing EpCAM as a marker. The separated and purified pituitary hormone-producing cells and the like exhibit superior pituitary hormone secreting ability by physiological stimulation of pituitary hormone secretion, and can be used for the treatment of the diseases relating to pituitary gland, using the cells. This application is based on a patent application No. 2020-065346 filed in Japan (filing date: March 31, 2020), the contents of which are incorporated in full herein.

## Claims

1. A method for separating pituitary hormone-producing cells and/or progenitor cells thereof, comprising a step of separating cells that express EpCAM from a cell aggregate comprising adenohypophysis and/or a precursor tissue thereof.

2. The method according to claim 1, wherein the cell aggregate comprises a hypothalamic neuroepithelial tissue.

3. The method according to claim 1 or 2, comprising a step of dispersing the cell aggregate to obtain a population of single cells before the step of separating the cells that express EpCAM.

4. The method according to any one of claims 1 to 3, comprising a step of shredding the cell aggregate comprising adenohypophysis and/or a precursor tissue thereof, or physically incising the cell aggregate comprising adenohypophysis and/or a precursor tissue thereof before the step of separating the cells that express EpCAM.

5. The method according to any one of claims 1 to 4, wherein the cell aggregate comprising adenohypophysis and/or a precursor tissue thereof is obtained by inducing differentiation of pluripotent stem cells.

6. The method according to claim 5, wherein the pluripotent stem cells are human induced pluripotent stem cells.

7. The method according to any one of claims 1 to 6, wherein the precursor tissue is pituitary placode and/or Rathke's pouch.

8. A method for producing pituitary hormone-producing cells and/or progenitor cells thereof, comprising a step of separating cells that express EpCAM from a cell aggregate comprising adenohypophysis and/or a precursor tissue thereof.

9. The method according to claim 8, comprising the following steps:
(A) a step of dispersing a cell aggregate comprising adenohypophysis and/or a precursor tissue thereof to obtain a population of single cells,
(B) a step of separating a population of cells that express EpCAM from the population, and
(C) a step of reaggregating the population obtained in the (B).

10. The method according to claim 8 or 9, wherein the pituitary hormone-producing cells and/or progenitor cells thereof are of a cell aggregate or a cell sheet.

11. The method according to claim 9 or 10, comprising a step of shredding the cell aggregate comprising adenohypophysis and/or a precursor tissue thereof, or physically incising the cell aggregate comprising adenohypophysis and/or a precursor tissue thereof before the (B) step of separating the cells that express EpCAM.

12. The method according to any one of claims 8 to 11,
wherein the pituitary hormone-producing cell is at least one type selected from the group consisting of growth hormone (GH)-producing cells, prolactin (PRL)-producing cells,
adrenocorticotropic hormone (ACTH)-producing cells, thyroid-stimulating hormone (TSH)-producing cells, follicle-stimulating hormone (FSH)-producing cells, and luteinizing hormone (LH)-producing cells.
